# EUROPEAN PATENT SPECIFICATION

(11) **EP 0 564 689 B1**
(45) Date of publication and mention of the grant of the patent: **23.07.1997**
(21) Application number: 92106281.6
(22) Date of filing: 10.04.1992
(51) Int. Cl.: A61K 39/112, A61K 39/116

(54) **Recombinant live vaccines against Gram-negative enteric pathogens**
Rekombinanter lebender Impfstoff gegen Gram-negative enterische Pathogene
Vaccin recombinant vivant contre des agents pathogène entériques Gram-négatifs

(43) Date of publication of application: 13.10.1993
(73) Proprietor: SCHWEIZERISCHES SERUM- & IMPFINSTITUT BERN, CH-3001 Bern (CH)
(72) Inventor: Viret, Jean-François, CH-3177 Laupen (CH); Cryz, Stanly J., Jr., CH-3176 Neuenegg (CH); Favre, Didier, CH-3176 Neuenegg (CH)
(74) Representative: VOSSIUS & PARTNER

(56) References cited:
- EP-A- 0 127 153
- EP-A- 0 250 614
- EP-A- 0 351 322
- WO-A-83/00437
- WO-A-89/12693
- WO-A-91/18092
- US-A- 3 856 935
- DATABASE WPIL Week 9019, Derwent Publications Ltd., London, GB; AN 90-139807

## Description

The present invention relates to recombinant live attenuated bacterial strains expressing O-serotype determinants of gram-negative enteric pathogens in a form covalently bound to a lipopolysaccharide (LPS) core, wherein the resulting LPS molecules are essentially indistinguishable from the ones present in the original pathogenic strains. Such recombinant carrier strains may be used as live vaccines for oral immunization against e.g. bacillary dysentery caused by virulent strains of Shigella spp.

Gram-negative enteric pathogens cause a variety of diseases, some of which may develop into acute diarrhoeal disease states and may eventually lead to the death of the infected individual. Examples of such diseases caused by said category of pathogens are shigellosis, typhoid fever, cholera, infections with enterotoxigenic and enteropathogenic Escherichia coli, and infections with Helicobacter pylori.

Shigellosis is an acute enteric disease caused by virulent strains of the species Shigella sonnei, Shigella boydii, Shigella dysenteriae, and Shigella flexneri. Upon ingestion, these organisms attach to and invade the lumen of the colon causing an acute inflammatory response. Disease symptoms include fever, tenesmus, cramps, and bloody stools often with mucous. Volunteer studies in which subjects who ingested S. sonnei and developed disease were latter re-challenged with S. sonnei showed that overt disease could induce a high level of immunity (Herrington et al., Vaccine **8:**353, 1990). This demonstrated the feasibility of developing a live attenuated Shigella vaccine. Additional findings (Cohen et al., J. Infect. Dis. **157:**1068-1071, 1988) suggest that the ability to induce an anti-LPS antibody response is critical for protection against shigellosis. At present, no vaccines are available for the prevention of shigellosis.

Shigella spp. O-serotypes are determined at the molecular level by so-called O-antigen structures, also termed O-chain or O-polysaccharide, which consist of polymerized sugar units anchored in the bacterial outer membrane as subcomponents of the lipopolysaccharide (LPS) molecules (Lüderitz et al., Curr. Top. in Membranes and Transport, **17:**79-149, 1982). O-serotypes are specifically determined by the chemical and structural composition of both the basic sugar unit and the polymerized O-polysaccharide. Genetically, enzymes involved in the biosynthesis of Shigella spp. O-antigen molecules are encoded within the rfb/rfc genetic locus which may be located on the bacterial chromosome and/or on a plasmid episome (Hale and Formal, Microb. Path., **1:**511-518. 1986). Within the rfb/rfc genetic locus, rfb genes encode enzymes involved in the biosynthesis of the basic sugar unit, whereas the rfc genes encode the O-antigen polymerase responsible for the polymerization of the basic unit to form high molecular weight O-antigen molecules which are required for immunogenicity.

In Enterobacteriaceae including Shigella spp. virulent strains the O-antigen is exposed on the cell surface in a form covalently bound to the core-lipid A moiety of LPS molecules.

Seven types of LPS cores have been structurally defined in Enterobacteriaceae, and named Ra, R1 to R4, K-12, and B (Lüderitz et al., Curr. Top. in Membranes and Transport **17:**79-149, 1982; Jansson et al., Eur. J. Biochem., **115:**571-577, 1981). S. sonnei LPS molecules have been shown to be of the R1 core type, whereas Salmonella spp. and the E. coli strains commonly used in cloning experiments are characterized by LPS of the Ra and K-12 types, respectively (Lüderitz et al., Curr. Top. in Membranes and Transport **17:**79-149, 1982). The chemical structure of LPS core(s) present in V. cholerae strains has not been determined yet. The genetic determinants for enterobacterial LPS core moieties are chromosomally encoded within the so-called rfa locus (Rick, p. 648-662, In Escherichia coli and Salmonella typhimurium cellular and molecular biology. F.C. Neidhardt (ed.). American Society of Microbiology, Washington, D.C., 1987).

The rfb and possibly the rfc genetic loci encoding S. sonnei O-antigen (which confers serospecificity) are present on a large (about 180 kilobases, kb) virulence plasmid (Kopecko et al., Infect. Immun. **29:**207-214, 1980). This plasmid also encodes a number of genetic loci which enhance the virulence of S. sonnei (Sansonetti et al., Infect. Immun. **34:**75-83, 1981). Formal and colleagues (Formal et al., Infect. Immun. **34:**746-750, 1981; U.S. Patent No. 4,632,830, 1986) have introduced this plasmid via conjugation into the Salmonella typhi vaccine strain TY21a (Germanier and Fürer, J. Infect. Dis. **131:**553-558, 1975; U.S. Patent No. 3,856,935, 1972) in an effort to a S. sonnei vaccine. This strain, termed 5076-1C, expressed the S. sonnei O-antigen as attached surface layer, not covalently attached to the core of S. typhi LPS (Seid et al., J. Biol. Chem. **259:**9028-9034, 1984), and perhaps other antigens and virulence determinants encoded by S. sonnei virulence plasmid.

Initial experiments, in which strain 5076-1C was orally administered to volunteers subsequently challenged with S. sonnei, showed 5076-1C able to confer a modest, but significant degree of protection against shigellosis (Black et al., J. Infect. Dis. **155:**1260-1265, 1987). However, subsequent trials of a similar design using 5 different lots of 5076-1C failed to show any evidence of protection against S. sonnei (Herrington et al., Vaccine **8:**353-357, 1990).

There are several possible explanations for this finding. First, it is known that the O-antigen moiety of LPS is highly immunogenic only when covalently attached to the core-lipid A moiety of LPS. Therefore, the presence of the S. sonnei O-antigen as a non-covalently attached cell surface layer on 5076-1C may not allow for routine induction of a protective immune response. Subsequent studies (Hartman et al., J. Clin. Microbiol. **29:**27-32, 1991) have revealed the 180 kb S. sonnei plasmid to be genetically unstable in 5076-1C, leading to the loss of the rfb/rfc locus and hence the inability to synthesize S. sonnei O-antigen. Finally, the expression of other genes encoded by the S. sonnei plasmid could adversely affect the growth and/or immunizing capacity of S. typhi strain TY21a.

In another study, the expression of V. cholerae O-antigen in S. typhi TY21a resulted in unbound O-antigen molecules, whereas expression in E. coli K-12 led to complete hybrid LPS molecules corresponding to V. cholerae O-antigen covalently bound to the K-12 core (Australian Patent No. AU-A-41023/89, 1988). In order to synthesize a complete LPS molecule carrying V. cholerae O-antigen in TY21a, the S. typhi rfa locus was replaced by a DNA region encompassing the corresponding rfa locus of E. coli K-12. As expected, the resulting strain, bearing in addition the V. cholerae rfb locus on a plasmid, expressed complete hybrid LPS consisting in V. cholerae O-antigen bound to K-12 type LPS. However, in the process of strain construction, not only the rfa locus but also up to 200 kb of adjacent genetic material, corresponding to about 5% of total chromosomal length, was replaced in TY21a. Such a large and uncontrolled manipulation may have affected the properties of the candidate vaccine strain in an unpredictable way.

Thus, the problem underlying the present invention was to provide hybrid live attenuated bacterial strains which stably express on their surface O-serotype determinants from gram-negative enteric pathogens in a form covalently bound to LPS molecules essentially indistinguishable from the ones present in the pathogenic strains to be vaccinated against, without affecting the properties of the candidate vaccine strain in any unpredictable way.

The solution to the above technical problem is achieved by providing the embodiments characterized in the claims.

Accordingly, the present invention relates to a recombinant live vaccine strain for the immunization against gram-negative enteric pathogens displaying the following features:
(a) it is a bacterial strain known to be safe and immunogenic when orally administered to humans;
(b) it carries a set of rfa, rfb and rfc genes coding for the enzymes involved in the biosynthesis of lipopolysaccharide core, preferably heterologous lipopolysaccharide core, and heterologous O-polysaccharide antigen;
(c) said set of genes is stably expressed by said carrier strain resulting in the formation of an intact immunogenic hybrid lipopolysaccharide composed of the lipid A from the carrier strain covalently attached to said preferably heterologous core and said heterologous O-polysaccharide, wherein said preferably heterologous core and said heterologous O-polysaccharide form an LPS molecule containing a polymerized O-polysaccharide and being essentially indistinguishable from the LPS molecule of the heterologous gram-negative enteric pathogen.

Said administration in item (a), supra, will usually be carried out with the bacteria being in a live form.

The evaluation of whether said heterologous O-polysaccharide is indistinguishable from the LPS molecule of said heterologous pathogen in item (c), supra, will usually be by physicochemical and/or immunological means.

The term "recombinant live vaccine strain" as used herein means a bacterial strain as defined in item (a) above which has been modified via recombinant DNA technology so that it carries and expresses at least one set of heterologous genes, wherein expression of said heterologous genes results in (a) new immunogenic property(ies) of said strain, allowing for the immunization against said gram-negative enteric pathogen when administered to humans.

The term "carrier strain" as used herein is intended to mean an attenuated bacterial strain carrying and expressing one or more foreign genetic determinants encoding protective antigens from a heterologous bacterium pathogenic for humans.

The term "being essentially indistinguishable from the LPS molecule of the heterologous gram-negative enteric pathogen" means an LPS molecule with a similar banding pattern when examined by polyacrylamide gel electrophoresis after appropriate treatment to visualize said banding, which in addition binds antibodies which are specific for the heterologous enteric pathogen and in addition elicits such antibodies when administered to an appropriate host.

In the case of virulent (Phase I) strains of S. sonnei the O-antigen moiety of LPS molecules consists in polymerized disaccharide units of two atypical sugars which are present on the cell surface in a form covalently bound to the R1-type LPS core-lipid A region (Form I LPS) (Kenne et al., Carbohydrate Res. **78:**119-126, 1980; Gamian and Romanowska, Eur. J. Biochem. **129:**105-109, 1982). On the other hand, efficient immunization against virulent strains of S. sonnei may require the immunizing antigen to be present on the surface of the live attenuated carrier cells in a form as close as possible to the one observed in natural virulent strains. As discussed above, previous evidence showed that the expression of the S. sonnei rfb/rfc genetic determinant from the whole S. sonnei virulence plasmid in the attenuated S. typhi/S. sonnei hybrid strain 5076-1C does not lead to the covalent attachment of the corresponding O-antigen onto the S. typhi Ra core structure (Seid et al., J. Biol. Chem. **259:**9028-9034, 1984). In the latter study, failure of getting complete O-antigen-bearing LPS molecules may have accounted for the weak immunization levels against virulent S. sonnei obtained with the hybrid strain.

It now appears that only certain combinations of rfa genes encoding the core region and rfb/rfc genes encoding the O-antigen lead to a stable expression of the O-antigen on the surface of a given cell. This surface expression is a result of the covalent attachment of the core and O-antigen moieties. Preferably, the resulting LPS has the same structure as in the original pathogen, being, for example, a strain of Shigella spp. (homologous core and O-antigen moieties). The minimal requirement for obtaining a hybrid polysaccharide displaying the feature of being essentially indistinguishable from the LPS molecule of the heterologous gram-negative pathogen is that all members of the respective set of genes encoding said LPS molecule are co-expressed in the same carrier strain.

Thus, in accordance with the present invention it will be necessary to obtain a set of genes which encodes different compatible components which form a functional LPS molecule. It is conceivable that part of said set of genes is a natural part of the carrier strain. Alternatively, it is possible that none of said genes is a natural part of said carrier strain. In either case the desirable genes to be introduced into said carrier strain will preferably be cloned from a suitable source. In the case of a specific Shigella O-serotype, for example, the corresponding rfb/rfc gene locus may be transferred from said Shigella strain to a suitable vaccine strain. If the vaccine strain carries the genes encoding a compatible core, no further cloning and transformation step may be necessary. Alternatively, if the vaccine strain does not carry said genes a further cloning step involving the original Shigella strain or another strain of the same or a different bacterial species carrying the genes encoding a compatible core will be necessary.

Upon cloning and transfer of a compatible set of genes into a suitable vaccine strain, said strain will express a complete LPS structure with the O-antigen of the pathogen displayed on its surface. As a result of this surface expression, the immune system of the vaccines is stimulated and a protective immune response, e.g. against S. sonnei, induced.

In a preferred embodiment of the vaccine strain of the present invention, said set of genes has been separated from non-relevant genetic information of the donor strain.

In the case of the genes encoding the S. sonnei O-antigen, the rfb/rfc locus must first be separated from other unrelated genes encoded by the 180 kb plasmid in order to stably and specifically express said locus. Separation of unrelated genes will, moreover, avoid adverse effects with respect to the growth and/or immunizing capacity of the carrier strain.

The preferred method of separating said locus from non-relevant genes is to produce a gene bank of the 180 kb plasmid in a suitable E. coli strain. Various methods of identifying recombinant clones expressing the O-antigen are known in the art. The method preferred herein is to use monoclonal or polyclonal antibodies specific for the S. sonnei O-antigen, preferably monoclonal antibodies (MAb).

A preferred scenario for the isolation of the core encoding genes would entail a DNA fragment carrying the rfa genetic determinant limited to the minimal size allowing for the synthesis of a heterologous LPS core able to covalently bind the Shigella O-antigen. Such a requirement is best satisfied via the cloning and characterization of the suitable rfa locus, i.e., defining the minimal DNA fragment necessary for its expression. A preferred situation would be one in which the cloned rfa locus expresses an LPS core having the same structure as the core to which the O-antigen is naturally coupled to. In the case of S. sonnei O-antigen, the preferred core would be of the R1 type.

Accordingly, in a preferred form, the present invention may include the cloning of chromosomal rfa loci encoding LPS core structures of the R1 type in order to even better simulate, in the recombinant vaccine strain, the LPS configuration present in virulent S. sonnei cells. Such rfa loci may be isolated from chromosomal gene banks of any available bacterial strains characterized by R1-type LPS. Preferred strains for this purpose may include, but are not limited to, E. coli R1 strains (rfa_{R1} locus) and S. sonnei strains (rfaₛₒₙₙₑᵢ locus). Further preferred strains may be the E. coli R1 strain HF4704 and the S. sonnei strain 53GII.

Suitable gene banks may be established in any E. coli K-12 strain commonly used for cloning purposes. However, an efficient screening methodology is essential for the identification of rfa clones expressing R1-type LPS. The preferred method may involve monoclonal antibodies (MAb) with the suitable specificity. Therefore, the isolation of MAb allowing for the specific identification of R1-type LPS is also part of the present invention.

In another preferred embodiment of the present invention, the carrier strain is an E. coli strain, a strain belonging to the genus Shigella, a S. typhi strain or a V. cholerae strain.

In a particularly preferred embodiment of the present invention, said strain is S. typhi TY21a or S. typhi CVD908.

The anti-typhoid live vaccine strain TY21a has been described in detail by Germanier and Fürer, J. Infect. Dis. 131 (1975), 553-558, and US-A-3,856,935 (1972). The aroC aroD deletion mutant strain CVD908 was described by Hone et al., Vaccine 9 (1991), 810-816.

In another particularly preferred embodiment of the present invention, said V. cholerae strain is V. cholerae CVD103, V. cholerae CVD103-HgR or V. cholerae CVD110. The serotype classical (Inaba) live vaccine strains CVD103 and CVD103-HgR have been described by Levine et al., Lancet 2 (1988), 467-470.

A further preferred embodiment of the present invention relates to a vaccine, wherein said set of heterologous genes are either present on a plasmid vector or stably integrated into the chromosome of the carrier strain at an integration site which is known to be non-essential for inducing a protective immune response by the carrier strain.

For cloning purposes and preliminary expression studies in recombinant vaccine strains, heterologous genes may most conveniently be propagated on plasmid vectors. However, stable maintenance of plasmid clones is frequently hard to achieve in recombinant strains, even when positive selection is allowed for a plasmid-encoded resistance marker. The complete stabilization of cloned genes may be performed via their integration into the chromosome of the relevant vaccine strain.

In the most favourable case, the integration of foreign genes into the carrier vaccine strain would occur via homologous recombination into a predefined region of the chromosome. Preferred targets for chromosomal integration are those genes or groups of genes, the activity of which is either already affected, or is known to be non essential for efficient immunization with the vaccine strain considered. Alternative preferred targets for integration are those genes, the inactivation of which leads to the attenuation of virulent strains related to the relevant vaccine strain.

In a particularly preferred embodiment of the present invention, said set of genes is integrated into an H₂S locus, the ilv locus, the viaB locus, or any of the aro loci of S. typhi.

Preferred target genes for chromosomal integration via homologous recombination into S. typhi TY21a may be a chromosomal locus involved in the production of H₂S (H₂S locus). Another target may be the ilv gene cluster (ilv locus) which encodes enzymes involved in the biosynthesis of the amino acids L-isoleucine (Ile) and L-valine (Val). Finally, a third target locus may be the viaB genetic locus encoding the Vi capsule antigen of S. typhi. Indeed, the vaccine strain TY21a has mutations affecting gene expression at all three loci (Silva et al., J. Infect. Dis. **155:**1077-1078, 1987). The preferred chromosomal integration sites for S. typhi CVD908 may be any one or more of the aro genetic loci as well as a locus involved in H₂S synthesis since aroC and aroD mutations also result in a lack of H₂S synthesis.

In another particularly preferred embodiment said set of genes is inserted into the hlyA locus of V. cholerae.

The target for chromosomal integration into V. cholerae strains CVD103, CVD103-HgR, and CVD110 may be hlyA, the genetic determinant for the V. cholerae hemolysin (hlyA locus). Indeed, disruption of this locus in CVD103-HgR via the integration of a mercury resistance genetic determinant was shown not to affect the immunogenicity of such live attenuated vaccine strains (Levine et al., Lancet **2:**467-470, 1988; Migasena et al., Infect. Immun. **57:**3261-3264, 1989).

The rfa, rfb and rfc genes are in another preferred embodiment of the present invention integrated in tandem into a single chromosomal integration site or independently integrated into individual integration sites.

These sites are preferably the ones which are non-essential for inducing a protective immune response.

In a further embodiment of the present invention, the rfa genes are derived from an E. coli strain and said rfb and rfc genes are derived from the same or different gram-negative enteric bacteria, preferably from the same or different gram-negative enteric pathogens.

In a particularly preferred embodiment of the present invention, said E. coli strain is characterized by an R1-type LPS core structure.

The R1-type structure is also present in Shigella species. Thus, a combination of the components of the LPS of the present invention may comprise an S. sonnei O-antigen in a form covalently bound to LPS of the R1 core type derived from E. coli. Accordingly, the heterologous S. sonnei rfb/rfc genes, on one hand, and the heterologous rfa genetic determinants for R1-type LPS core structures from a different source organism, on the other hand, may be co-expressed in the desired carrier strain. Carrier strains for the expression of complete S. sonnei Form I type LPS molecules may include, without limitation, S. typhi or V. cholerae live attenuated vaccine strains. Preferred S. typhi carrier strain may be the anti-typhoid live vaccine strain TY21a (Germanier and Fürer, ibid.; U.S. Patent No. 3,856,935) or the aroC aroD deletion mutant strain CVD908 (Hone et al., ibid.), whereas V. cholerae carrier strains may be one of the serotype classical (Inaba) live vaccine strains CVD103 or CVD103-HgR (Levine et al., ibid.). An alternative V. cholerae carrier strain may be the serotype Eltor (Ogawa) live vaccine strain CVD110.

In another preferred embodiment of the present invention, said gram-negative enteric pathogens are Shigella sonnei, Shigella boydii, Shigella dysenteriae or Shigella flexneri, preferably Shigella flexneri serotpye IIa. The most preferred strains are from the species Shigella sonnei.

Another embodiment of the present invention relates to a vaccine strain wherein said rfa, rfb and rfc genes are all derived from said gram-negative enteric pathogen, preferably from Shigella sonnei, Shigella boydii, Shigella dysenteriae or Shigella flexneri, preferably from Shigella flexneri serotype IIa, and most preferably from Shigella sonnei.

A further object of the present invention is to provide a live vaccine for the immunization against gram-negative enteric pathogens comprising a strain of the invention, optionally in admixture with a pharmaceutically acceptable excipient and/or buffer.

The vaccine of the invention may successfully be used for the prevention of any disease caused by enteric pathogens. Examples of such diseases are diseases caused by S. sonnei, S. boydii, S.dysenteriae, S.flexneri, Salmonella typhi, Salmonella enterititis, E. coli being enteropathogenic, enterohemorrhagic, enteroenvasive or enterotoxinogenic, V. cholerae, Helicobacter pylori, and Clostridium deficile. Such an invention may also prevent enteric diseases caused by Giardia lambia and Enteroamoeba histolyticum.

The vaccine of the invention is formulated according to standard procedures known in the art which consist of cultivating said vaccine on an appropriate medium, collection of said culture, mixing such cultured bacteria with an appropriate cryoprotective medium, lyophilization of said mixture under conditions which will yield ≥0.1% of the initial bacterial count after subsequent reconstitution, and packaging into a container, said container preferably being a gelatin capsule, an enteric-coated gelatin capsule, glass or plastic vials, or an aluminum foil sachet. Said formulation is designed to facilitate the passage in a viable state of the vaccine through the stomach gastric acid barrier.

The vaccine will usually contain as the effective agent 1x10⁵-1x10¹⁰ viable bacteria, the preferred range being 1x10⁶-10⁹ colony forming units per dose.

The effective agent will preferably, but not necessarily, be admixed with a pharmaceutically acceptable excipient and/or buffer and/or any delivery system designed to deliver said vaccine strain in a viable state to the intestinal tract. Examples of such excipients are a sugar, specifically glucose, lactose, sucrose or sorbitol, a mixture of amino acids, preferably a digest or hydrolysate of casein, tryptone, peptone or heart-infusion preparation, or a combination of a sugar-amino acid mixture, with or without a sweetening and/or flavoric agent, with or without an antioxidant, preferably ascorbic or citric acid.

Examples of said buffers are sodium carbonate, alone or in combination with a pharmaceutically acceptable acid, preferably ascorbic acid, combined in such proportion that when appropriately reconstituted, it yields a solution with a pH of 3 to 8, preferentially pH 6 to 8.

Said vaccine is administered to vaccines by oral ingestion of an enteric coated capsule, ingestion of the vaccine organisms either after ingestion or ingested simultaneously with a buffer designed to neutralize gastric acid.

The preferred route of administration is the oral route.

### EXAMPLES

### Example 1

### Isolation of hybridoma cell lines secreting monoclonal antibodies (MAbs) specific for either the O-polysaccharide or the core moiety of S. sonnei LPS molecules

**Bacterial strains and plasmids.** Bacterial strains used to immunize mice and for the characterization of LPS structures with MAbs are listed in Table 1.

**Production of MAbs.** In order to isolate hybridoma cell lines secreting the suitable MAbs, female BALB/C mice were repeatedly immunized intraperitoneally with about 5.10⁸ heat-killed (1 hour, 60°C) cells of S. sonnei Phase I strain 53GI resuspended in 500 µl of phosphate-buffered saline (PBS). Spleen immune cells were fused with the non-producing myeloma cell line X63-Ag8.653 (Kearney et al., **123:**1548-1550, 1979) as previously described (Lang et al., Infect. Immun. **57:**2660-2665, 1989). Hybridoma culture supernatants were assayed for the production of anti-LPS antibodies by ELISA assays using purified LPS (see below). Supernatants were screened in parallel for antibody binding against LPS from S. sonnei 53GI and its rough variant 53GII. Positive hybridomas were cloned by limiting dilution. Class and subclass determination of the MAbs were performed by ELISA (see below) using a mouse typer kit (Bio-Rad Lab., Richmond, CA, USA).

**Preparation of LPS.** LPS were isolated by the hot phenol method of Westphal et al. (Z. Naturforsch. **TeilB:**148-155, 1952) and further purified by incubation in presence of ribonuclease, deoxyribonuclease and pronase treatments followed by repeated ultracentrifugation rounds as previously reported (maxiprep method) (Cryz et al., Infect. Immun. **44:**508-513, 1984). Purified LPS of rough variants of E. coli (R4 core type) and S. typhimurium (Rc and Rd1 core types) were purchased from Biocarb Chemicals, Lund, Sweden. For fast analysis purposes, LPS were extracted from late exponential or stationary liquid cultures using a rapid small-scale mini-prep method (Hitchcock and Brown, J. Bacteriol. **154:**269-277, 1983).

**SDS-PAGE and immunoblots.** Analysis of LPS preparations by electrophoresis on sodium dodecyl Sulfate-polyacrylamide gels (SDS-PAGE) was performed essentially as described by Laemmli (Nature **227:**680-685, 1970) on either 15% or 7.5-20 % acrylamide gels. LPS molecules were then silver stained using the procedure of Hitchcock and Brown (Hitchcock and Brown, J. Bacteriol. **154:**269-277, 1983).

For immunoblot analysis, LPS separated on gel were electroblotted onto nitrocellulose filters (Towbin et al., Proc. Natl. Acad. Sci. USA **76:**4350-4354, 1979). Blocking of the filters, incubation with antibodies and enzymatic detection of the immunoreactive material were essentially as described previously (Lang et al., Infect. Immun. **57:**2660-2665, 1989).

For immunodot analysis of whole bacterial cells, the latter were washed, resuspended in one volume of PBS, and 5 µl spotted onto nitrocellulose filters. The spots were allowed to dry at room temperature and were then processed as for LPS immunoblots, starting from the blocking step, except that incubations with the first and second antibodies were each for 30 minutes at room temperature.

**ELISA.** Binding assays were performed as described (Bruderer et al., J. Immunol. Methods **133:**263-268, 1990) with the exception that for coating LPS was incubated for 2 hours at 37°C, followed by an overnight incubation at 4°C. Peroxidase conjugated affinity isolated goat anti-mouse IgM antibodies (Tago, Burlingame, USA, or Sigma Chemicals, St. Louis, USA) were used as developing antibodies.

**Characterization of the MAb-producing cell lines.** Three hybridoma cell lines secreting monoclonal IgM antibodies (MAb) directed against S. sonnei LPS molecules were generated from BALB/c mice immunized with heat-killed whole cells of S. sonnei strain 53GI. The binding of these MAbs to LPS isolated from smooth (Phase I) and rough (Phase II) variants of S. sonnei strain 53G is shown in Table 2. MAb Sh5S and Sh11S exhibited high ELISA titers for the high molecular weight (hmw) Form I LPS consisting of core-lipid A and O-polysaccharide moieties, but did not react with the low molecular weight Form II LPS lacking the O-polysaccharide. In contrast, MAb Sh9R reacted with both kinds of molecules with a preference for the Form II LPS. Consistently, immunoblot analysis revealed that Sh5S and Sh11S bind to O-polysaccharide bearing LPS, whereas Sh9R specifically reacts with the core-lipid A moiety.

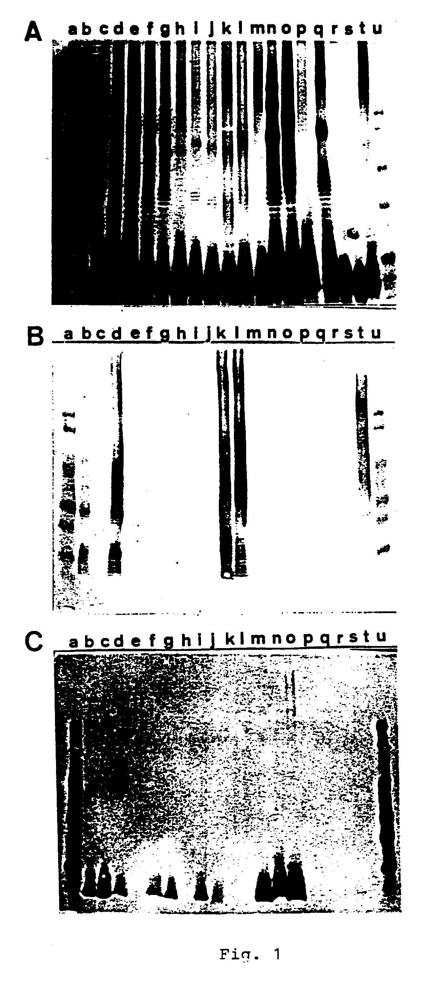

The specificity of Sh9R and Sh5S MAb was further examined by immunoblot analysis of LPS isolated from a panel of bacterial strains of known O-antigen specificity and LPS core structure. As shown in Figure 1, the O-polysaccharide specific MAb Sh5S (Fig. 1B) recognized LPS molecules from different Phase I strains of S. sonnei (lanes b and d) as well as from strains M51 and C27 of P. shigelloides (lanes k and l) reported to belong to the same O-serotype as S. sonnei (Basu et al., FEMS Microbiol. letters **28:**7-10, 1985; Rauss et al., Acta microbiol. Acad. Sci. hung. **17:**157-166, 1970). For all those strains only high molecular weight (smooth) LPS molecules were detected on the blot. Furthermore, O-polysaccharide determinants expressed by the S. typhi hybrid strain TY37 (lane t) were also recognized by MAb Sh5S although no hmw Phase I-like LPS could be detected by silver staining. Strain TY37 was constructed by introducing via conjugation the Tn5-tagged S. sonnei virulence plasmid pWR105 from strain 482-79(pWR105) into the S. typhi live oral vaccine strain TY21a, using the mobilizing replicon R386. Thus, TY37 contains the whole S. sonnei virulence plasmid, thereby being essentially equivalent to the hybrid strain 5076-1C (Formal et al., Infect. Immun. **34:**746-750, 1981; Formal et al., U.S. Patent No. 4,632,830) with the further advantage that the virulence plasmid can be maintained more stably via selection for the transposon Tn5-encoded kanamycin resistance marker. According to the results presented here for TY37 and to published data for 5076-1C (Seid et al., J. Biol. Chem. **259:**9028-9034, 1984), both strains present similar properties as far as S. sonnei O-antigen expression is concerned.

Using Sh9R to probe the same LPS preparations (Fig. 1C), only those strains reported to have LPS molecules with an R1-type core structure could be recognized, namely S. sonnei (lanes b to d), S. boydii type 3 (lane g), S. flexneri type 6 (lanes i and j), a control E. coli R1 strain (HF4704, lane m) and an E. coli O9 isolate (lane o). To our knowledge, the LPS core structure of S. boydii type 1 has not been described yet. Since the reference strain ATCC 9207 (lane f) also gave a positive signal, we conclude that its LPS is of the R1 type. Two possible LPS core structures, R1 and R2, were documented for E. coli strains of the 08 serotype (Schmidt et al., Eur. J. Biochem. **10:**501-510, 1969; Schmidt et al., Eur. J. Biochem. **16:**382-392, 1970); the pattern observed for 08 strain ATCC 23504 (lane n) indicates that it bears the R1-type LPS. Two independent strains of S. flexneri type 6 were also tested. Although similar amounts of LPS of both strains were loaded onto the gel (Fig.1A, lanes i and j), one of those (ATCC 14025) provided a weaker signal with Sh9R MAb (lane j) whereas the second strain (S323, lane i), and other clinical isolates from the same source (not shown), gave a higher signal. These observations may indicate some variability in the core-lipid A region of different S. flexneri type 6 isolates. Finally, Sh9R MAb did not recognize LPS from P. shigelloides strains M51 and C27 (lanes k and l) although serological and chemical analyses suggested that they may be of the R1 core type (Table 1). Binding activity of Sh9R MAb was detected neither with Ra, R2, R3, R4, nor with K-12 LPS. In addition, binding was not observed with V. cholerae serotype O1 LPS of either the Inaba or the Ogawa subtype (data not shown).

The reactivity of the core-specific MAb Sh9R was also examined in an immunoblot of LPS preparations from clinical isolates of E. coli and Pseudomonas aeruginosa with O-serotypes among the most frequently encountered in nosocomial cases of bacterial sepsis (Cryz, p.317-351. In R. Germanier, ed., Academic Press, Inc., Orlando, USA, 1984; Cryz, p.186-195. In S.J. Cryz, Jr, ed., Pergamon Press, Inc., New York, USA, 1990; Orskov and Orskov, Methods in Microbiol. **14:**43--112, 1984) (Table 3). Among the E. coli strains, seven out of ten (with 01, 02, 06, 07, 018, 025, and 075 serotypes) gave a strong reaction indicating that despite their wide range of O-antigen serotypes they bear LPS with a R1-like core structure. In contrast, LPS preparations from nine clinical isolates of P. aeruginosa failed to react with Sh9R MAb.

### Example 2

### Cloning and physical mapping of S. sonnei rfb/rfc genes

**Preparation of a gene bank of the S. sonnei virulence plasmid.** As a first step towards the cloning of the S. sonnei rfb/rfc genetic locus, a gene bank of the large virulence plasmid was prepared in the low copy number cosmid vector pLAFR-5. Plasmid DNA purified from S. sonnei strain 482-79(pWR105) (Table 1) was partially digested with the restriction enzyme Sau3AI and size selected 20 to 30 kilobases (kb) long DNA fragments were cloned in the BamHI site of pLAFR-5. In vitro packaging of DNA was done using a commercial kit (Gigapack^{R} II Plus, Stratagene, La Jolla, CA, USA) accordingly to the manufacturer's instructions. Packaged DNA was transfected into E. coli K-12 strain HB101 (Table 4) and transfected cells selected on tetracyclin (12.5 mg/ml) plates. Resistant colonies were resuspended in liquid medium, pooled and aliquots kept frozen in presence of 40% glycerol at - 70°C.

**Screening of the cosmid bank for colonies expressing S. sonnei O-antigen.** One frozen aliquot of the cosmid bank was diluted and plated for isolated colonies on tetracyclin plates. Grown colonies were transferred onto nitrocellulose membrane filters (Schleicher and Schüell, Feldbach, Switzerland). Filters were then processed for immunodetection according to published protocols (Sambrook et al. Molecular Cloning, 2^{nd} edition, Cold Spring Harbor Laboratory Press, Cold Spring Harbor, USA, 1989). Probing the filters with the S. sonnei O-antigen-specific MAb SH5S resulted in the isolation of 15 independent positive clones. After further propagation on selective plates, all the clones were still strongly reacting with Sh5S and the cells could be agglutinated in the presence of an anti-S. sonnei rabbit polyclonal antiserum pre-adsorbed against S. sonnei Phase II cells. Four clones (pSSVI108, 109, 110, 111) were further characterized.

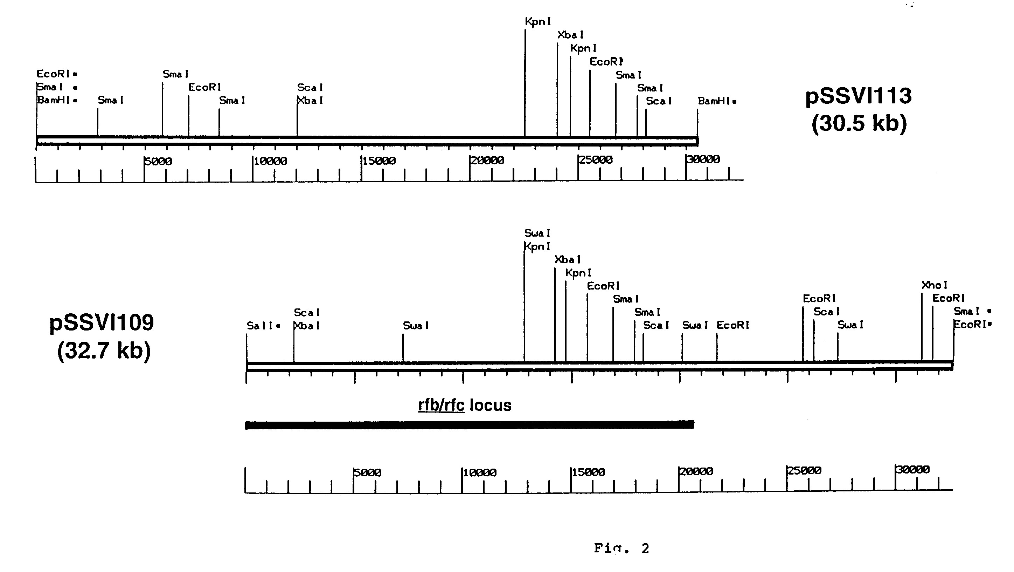

**Restriction analysis of positive clones.** Digestion of candidate rfb/rfc clones with restriction enzymes gave almost identical patterns for clones pSSVI109, 110, and 111 whereas clone pSSVI108 only partially overlapped with the other ones. The S. sonnei rfb/rfc gene cluster could be subcloned on a BamHI fragment of about 30 kb, encompassing almost the whole pSSVI108 insert, into the BamHI site of the low copy number vector pGB2. The resulting clones, pSSVI113 and pSSVI114, bear the 30 kb BamHI fragment in either orientations. As revealed in Figure 2, the inserts present in clones pSSVI113 and pSSVI109 overlap by about 20 kb. However, a DNA segment of about 10 kb on one side of pSSVI113/114 insert (left end of pSSVI113 insert as depicted in Fig. 2) is absent in pSSVI109. The same DNA segment is also missing in other independently isolated rfb cosmid clones, such as pSSVI110 and 111 (data not shown). Finally, a critical examination of the restriction maps of Fig. 2 clearly shows that the DNA segments relevant to the present invention differ from the ones described in a previous study by Yoshida et al. (J. Gen. Microbiol. **137:**867-874, 1991).

**Isolation of the virulence plasmid minimal replicon.** The DNA region from the S. sonnei large virulence plasmid which is unique to pSSVI113 could be excised from the latter on an EcoRI fragment of about 7 kb (see Fig. 2). Interestingly, when cloned alone into the EcoRI site of the suicide vector pGP704, giving rise to plasmid pSSVI162, this 7 kb fragment was able to suppress the pGP704 replication deficiency in non-permissive hosts, such as E. coli K-12 strain DH5a. The same suppression effect was observed after subcloning of a 3 kb SmaI fragment internal to the 7 kb EcoRI fragment of pSSVI113 into the pGP704 SmaI site (plasmid pSSVI163). These observations suggested that the about 30 kb BamHI insert of plasmids pSSVI113 and pSSVI114 contains, in addition to the S. sonnei rfb/rfc locus, a segment of DNA which is able to self-replicate. In order to confirm this, the isolated BamHI insert of pSSVI114 was ligated to a BamHI DNA fragment of about 1.3 kb from plasmid pWW84 encoding resistance to the antibiotics kanamycin. The ligation mixture was transformed into E. coli DH5a and transformed cells selected on plates with 50 mg/ml of kanamycin. Thereby, a kanamycin-resistant (KmR) clone was isolated, named pSSVI161, which, by restriction and physiological analysis was shown to contain no pGB2 vector sequences. Plasmid pSSVI161 replicates at a reduced copy number as compared to pSSVI114. Cells bearing pSSVI161 expressed high amounts of S. sonnei O-antigen detectable with the S. sonnei O-antigen specific MAb SH5S.

We conclude then that the BamHI DNA fragment present in plasmids pSSVI113 and pSSVI114 contains an internal DNA sequence which behaves as an autonomous replicon. The relevant sequence could be mapped to the left end of the insert of pSSVI113, as shown in Fig. 2, within a 7 kb EcoRI fragment and a 3 kb SmaI fragment internal thereof. This DNA region may encompass the origin of replication of S. sonnei large virulence plasmid that we propose to call oriVIR. This would imply that the rfb/rfc locus maps extremely close to oriVIR on the S. sonnei virulence plasmid.

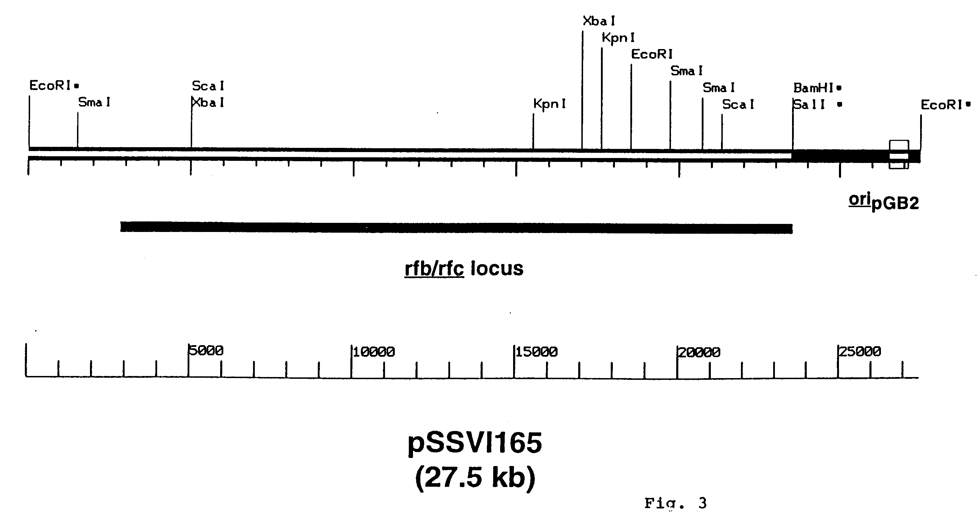

**Subcloning of S. sonnei rfb/rfc genes independently of the adjacent oriVIR fragment.** Knowing the position of oriVIR within the insert of pSSVI113 and pSSVI114, we then attempted the subcloning of the rfb/rfc locus independently of the adjacent internal replicon. For this purpose, pSSVI113 was partially digested with EcoRI, self-ligated and the ligation mixture transformed into E. coli DH5a with selection for the pGB2-encoded spectinomycin-resistance marker. We could thereby isolate transformants which stably expressed S. sonnei O-antigen while lacking the 7 kb oriVIR-bearing EcoRI fragment. Plasmid DNA isolated from one transformant colony was shown to contain only the right part of pSSVI113 insert (18 kb + 5 kb EcoRI fragments, see Fig. 2) linked to pGB2. The corresponding clone (pSSVI165) has a total size of 27.5 kb, including vector sequence (Fig. 3).

### Example 3

### Cloning and physical mapping of E. coli R1 rfa genes

**Preparation of an E. coli R1 chromosomal bank.** The genetic determinants for the biosynthesis of LPS molecules of the R1 core type were cloned from a chromosomal gene bank of E. coli R1 strain HF4704 (Table 1). Purified chromosomal DNA was partially digested with Sau3AI restriction enzyme and size-selected (20-30 kb) fragments from a sucrose gradient were ligated into cosmid vector pLAFR-5. In vitro packaging of ligated DNA and transfection of E. coli K-12 strain HB101 was performed as described in Example 2. The bank was kept frozen in presence of 40% glycerol at -70°C.

**Screening of the bank for the expression of R1-type LPS.** Transfected colonies grown out from the frozen bank were treated as described in Example 2, except that probing was using the R1 core-specific MAb Sh9R. Initially, two clones were isolated, named pSSVI106 and 107, which expressed R1-type LPS on the cell surface.

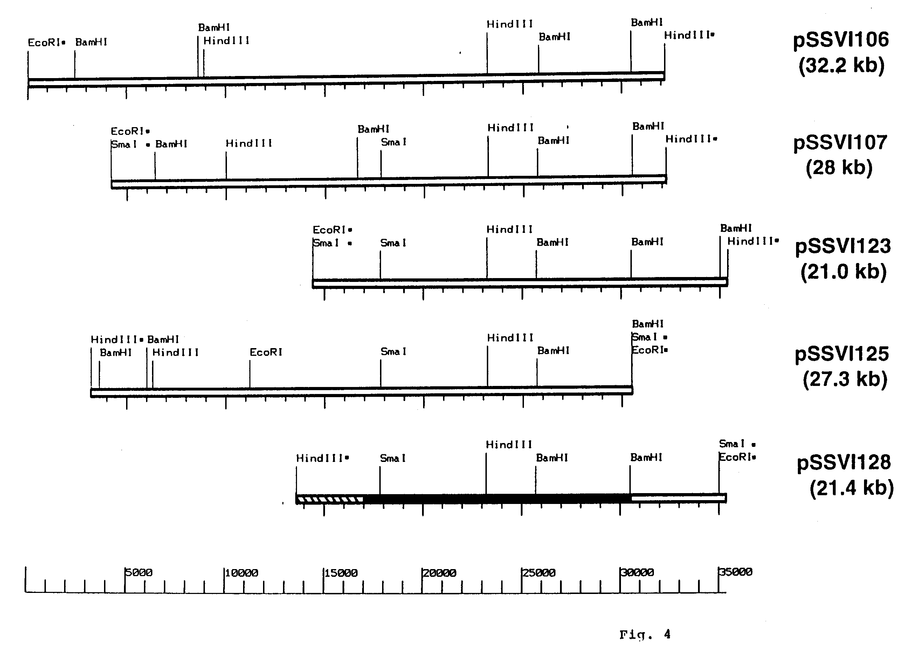

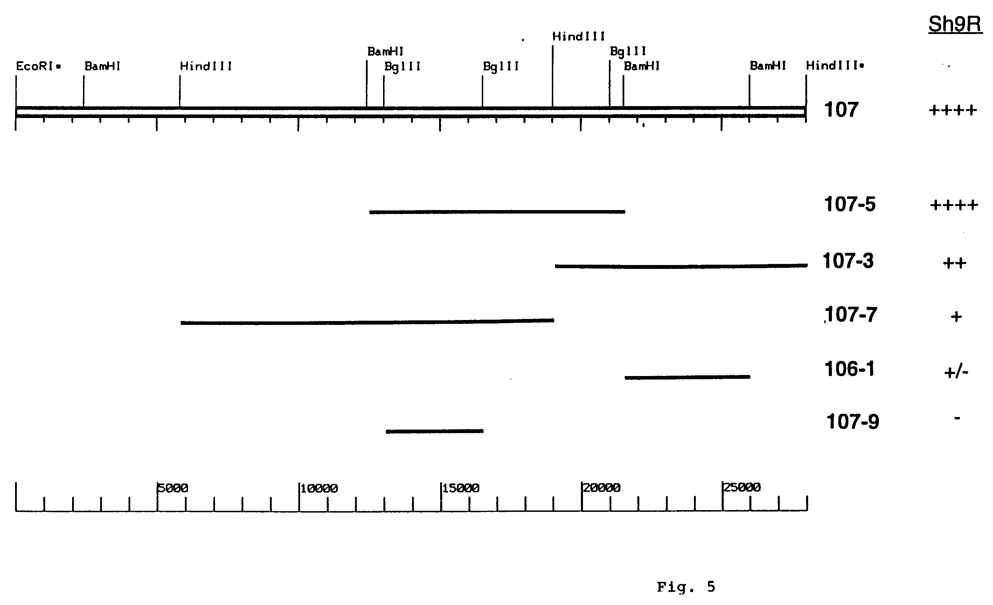

**Restriction mapping of the rfa**_{**R1**} **clones pSSVI106 and pSSVI107.** Physical maps of plasmids pSSVI106 and 107 carrying the rfa_{R1} genes were constructed with respect to restriction enzymes BamHI and HindIII (Fig. 4). Comparison of these maps and Southern hybridization analysis (Southern, J. Mol. Biol. **98:**503-517, 1975) of plasmid DNA preparations using subfragments of pSSVI106 and pSSVI107 as probes revealed a 4.5 kb BamHI fragment and the adjacent 2.5 kb BamHI-HindIII one as the only common fragments. Accordingly, sub-cloning of the 4.5 kb BamHI fragment alone from plasmid pSSVI106 into vector pCL1920 leads to plasmid pSSVI106-1 which expresses an epitope recognized by MAb Sh9R, although less strongly than the parent clones. This result indicated that some information was lost after subcloning. In order to better define this loss, BamHI (pSSVI107-5, pSSVI107-6), HindIII (pSSVI107-3, pSSVI107-4, pSSVI107-7) and BglII (pSSVI107-9) subclones were isolated from pSSVI107. The DNA fragments of pSSVI107 present in those plasmids and the degree of reactivity of E. coli K-12 cells bearing each subclone with MAb Sh9R are shown in detail in Fig. 5. Response of the subclones to Sh9R varied from nil to full. Interestingly, the strongest response did not come from the association of the fragments shared between pSSVI106 and 107, but from pSSVI107-5, a clone which carries a 9 kb BamHI subfragment from pSSVI107 which overlaps the 2.5 kb, but not the 4.5 kb fragment. Southern analysis indicated that the 9 kb fragment shares extensive homology with a 17 kb BamHI fragment from pSSVI106, although they do not seem to overlap. Possible explanations for this discrepancy included rearrangements or chimeric composition of at least one of the clones. To clarify the latter point, the 4.5, 9, and 17 kb BamHI fragments were further hybridized to total digests of E. coli R1 chromosomal DNA. Both the 4.5 and 17 kb bands were identified at their expected sizes whereas the 9 kb band hybridized to a 17 kb fragment. This result indicates that part of the 9 kb BamHI fragment present in pSSVI107-5 must correspond to the distal part of the 17 BamHI kb fragment of pSSVI106. Thus, some DNA rearrangements may have happened within clones pSSVI106 and/or pSSVI107, either by deletion of an extensive internal fragment or from the ligation of one or more non-adjacent smaller fragment(s) during the preparation of the gene bank.

To dispel any doubts, further rfa clones were isolated from the E. coli R1 chromosomal bank and named pSSVI123 to 131. Out of nine clones analyzed, seven were found to have a similar BamHI-HindIII restriction pattern. The 4.5 kb BamHI and 2.5 kb BamHI-HindIII fragments common to pSSVI106 and 107 are always present in the new clones, indicating that they should play a central role in the expression of the rfa_{R1} core. The restriction maps of three of the new clones, pSSVI123, pSSVI125 and pSSVI128, are shown in Fig. 4. Mapping data, together with evidences from expression studies presented below (Examples 6 and 7), allow to define an overlap region of about 13.5 kb (black area in the map of pSSVI128, Fig. 4) which may be close to the minimal DNA length required for full expression of the R1 LPS core in E. coli K-12. Outside this region clones, such as pSSVI106 and pSSVI107, may have undergone some rearrangements. However, an additional DNA segment of about 3.5 kb (hatched area in the map of pSSVI128, Fig. 4) seems to be required for full expression of the R1 core in other strains, such as, e.g., S. typhi and V. cholerae. Thus, the whole rfa_{R1} locus defined here has a size of about 17 kb (black and hatched area in Fig. 4).

**Southern hybridization analysis of the rfa locus from different enterobacterial species with LPS of the R1 core type.** The degree of sequence similarity between the cloned rfa_{R1} locus and corresponding chromosomal DNA sequences present in different enterobacterial strains with reported R1-type LPS (Example 1 and Table 1) was examined using the 4.5 kb and 9 kb BamHI fragments of pSSVI107 and the 17 kb BamHI fragment of pSSVI106 as probes for Southern analysis. Results showed sequence similarity to chromosomal DNA isolated from E. coli O9, S. sonnei 53GII, S. flexneri serotype 6 (S323), S. flexneri serotype 6 (ATCC 12025), and S. boydii, serotypes 1 and 3. There was no detectable similarity to E. coli C600 and P. shigelloides C27 chromosomal DNA. Thus, there is a perfect match between data obtained by DNA:DNA hybridization using rfa_{R1}-specific probes and the immunodetection of R1-type LPS with the R1 core specific MAb SH9R (see Example 1). However, the interspecies similarity of rfa genes is apparently not complete since the target fragment sizes for one given probe showed substantial variations, revealing a certain degree of restriction polymorphism between bacterial species with rfa genes encoding R1-type LPS (data not shown).

### Example 4

### Cloning and physical mapping of S. sonnei rfa genes

**Preparation of a S. sonnei chromosomal gene bank.** In order to clone the S. sonnei rfa (rfaₛₒₙₙₑᵢ) locus encoding R1-type LPS core structure, a cosmid bank of S. sonnei chromosomal DNA was prepared in cosmid vector pLAFR-5. Purified chromosomal DNA from S. sonnei 53GII (Table 1) was partially digested with Sau3AI and size-selected (30 to 40 kb) fragments ligated into the BamHI site of pLAFR-5. In vitro packaging and transfection of E. coli K-12 strain HB101 were as described in Example 2.

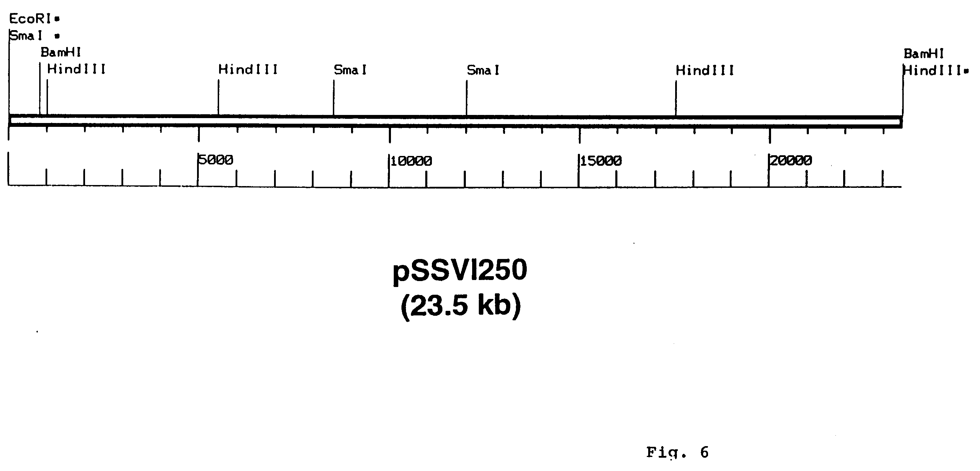

**Screening of the gene bank for clones expressing rfa**_{**sonnei**} **genes and restriction mapping of some isolated clones.** Transfectant colonies grown out on selective plates from the frozen bank were screened for expression of the rfa genes using Sh9R MAb as described in Example 2. Six out of sixteen isolated positive clones, named pSSVI232, pSSVI233, pSSVI234, pSSVI249, pSSVI250, and pSSVI251, were mapped with respect to EcoRI, BamHI, and HindIII restriction enzymes and shown to carry widely overlapping DNA inserts. As an example, a restriction map of clone pSSVI250 is shown in Figure 6.

### Example 5

### Expression studies of S. sonnei rfb/rfc genes in heterologous genetic backgrounds including live vaccine strains

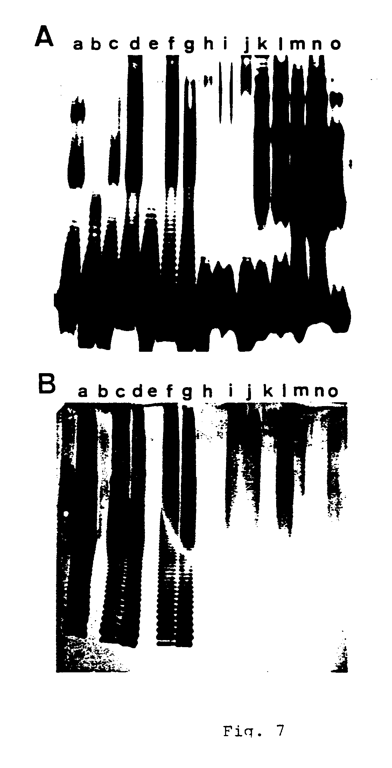

Selected S. sonnei rfb/rfc clones were transferred via electrotransformation or conjugation/mobilization methodologies into different strains and the expression of S. sonnei O-antigen was examined using MAb Sh5S for the immunodot blot analysis of whole cells and in immunoblot experiments of LPS preparations as described in Example 1. Results of one typical immunoblot experiment are presented in Fig. 7 for different strains (Table 1 and 4) bearing the rfb/rfc cosmid clone pSSVI109.

In both S. sonnei Phase II variant 53GII and E. coli K-12 strains (lanes d, e, g, and h), the expression of rfb/rfc genetic determinant resulted in the synthesis of large amounts of high molecular weight (hmw) Form I type LPS detectable as a ladder both by SDS-PAGE analysis and silver staining of LPS minipreps and on the immunoblot using MAb Sh5S. Thus, the expression of the cloned rfb/rfc genes in the above strains resulted in the same LPS pattern as in S. sonnei Phase I strains (lane b). This shows that the S. sonnei O-antigen expressed from clone pSSVI109 may be coupled not only onto the homologous R1 LPS core type but also onto the K-12 core. Strong evidence for covalent linkage of the O-antigen to the K-12 core came out of the fact that LPS maxipreps (hot phenol method, see Example 1) provided the same pattern as LPS minipreps (lanes g and h).

In contrast, as observed for the S. typhi/S. sonnei hybrid strain TY37 (see Example 1), no ladder of silver stained hmw LPS could be correlated with the expression of the rfb/rfc genes in S. typhi strain TY21a (lane j). S. sonnei O-antigen molecules produced in the latter strain were detected as a smear with a few faint discrete bands in the intermediate molecular weight region when probed with Sh5S MAb in an immunoblot. Moreover, most of this material was lost during LPS purification via the hot phenol method (lane k) indicating that it is only loosely bound to the cell surface. Finally, expression of rfb genes in V. cholerae strain CVD103-HgR (classical Inaba) (lanes m and n) also resulted in the production of S. sonnei O-antigen in a form unbound to LPS core, although in larger amounts as in TY21a. Good levels of unbound S. sonnei O-antigen were also observed in V. cholerae strain CVD110 (Eltor Ogawa) (lane o).

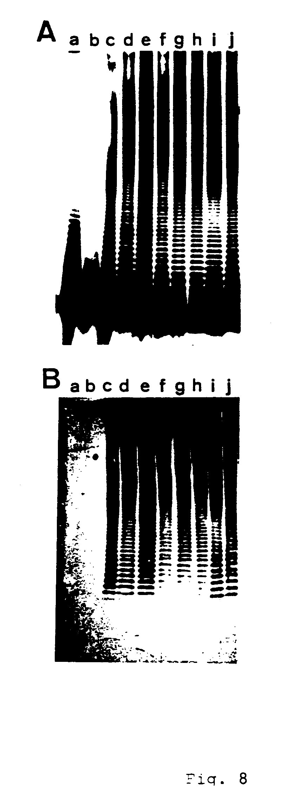

The detection of high amounts of Sh5S-reactive LPS in preparations of E. coli K-12 and S. sonnei strains bearing clone pSSVI109 demonstrates that the cloned genes actually correspond to the S. sonnei rfb locus responsible for the biosynthesis and assembly of the S. sonnei O-antigen disaccharide unit structure. In addition, the fact that a large part of the O-antigen produced is present in the form of hmw Form I type LPS is compatible with the presence within the cloned DNA fragment of the rfc locus encoding the O-antigen polymerase. From the above data, however, a complementation effect by a chromosomally encoded rfc locus cannot be excluded. Therefore, LPS patterns associated with different clones and subclones were examined in the E. coli K-12 strain SF874 which presents a chromosomal deletion that removed the whole E. coli K-12 rfb/rfc locus. As shown in Fig. 8, cosmid clone pSSVI109 and subclones pSSVI114, 161, and 165 provided the same LPS pattern in SF874 as in the control E. coli K-12 strain. These results show that all plasmid tested contain the complete genetic information necessary and sufficient for the synthesis of long chain S. sonnei O-antigen molecules which are present as Form I type LPS.

### Example 6

### Expression studies of E. coli R1 and S. sonnei rfa genes in heterologous genetic backgrounds including live vaccine strains

A detailed study of the expression of some of the rfa_{R1} clones described in Example 3 was performed in order to pin-point the minimal DNA length necessary and sufficient for the production of LPS molecules of the R1 core chemotype. Expression levels and types of LPS produced were determined by immunodot blot analysis of whole cells and by immunoblot analysis of LPS preparations using the R1 core-specific MAb Sh9R.

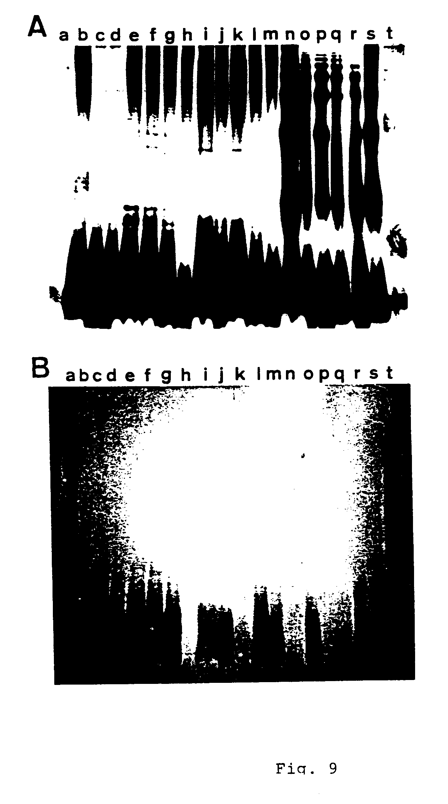

As illustrated for some clones in Fig. 9, and summarized in Table 5, the requirements for producing a R1-type LPS core are similar but not identical in E. coli K-12 and S. typhi, which are characterized by structurally related indigeneous LPS core structures (Lüderitz et al., Curr. Top. in Membranes and Transport **17:**79-149, 1982; Jansson et al., Eur. J. Bio-chem., **115:**571-577, 1981), whereas V. cholerae has more stringent requirements. Most of the original plasmid clones (pSSVI106, pSSVI107, and pSSVI123 to 130) expressed strongly reacting LPS cores in S. typhi (with the exception of pSSVI127 and pSSVI130 giving a low signal). On the other hand, subclone pSSVI107-3 gave an intermediate signal in E. coli K-12 but was negative in S. typhi. In V. cholerae CVD103-HgR, only some of the cosmid clones gave a strong signal; pSSVI106 showed a weaker response, and pSSVI107 was even weaker probably due to its instability. Finally, subclone pSSVI107-5 was negative in CVD103-HgR while being strongly positive in both E. coli K-12 and S. typhi.

The same analyses were performed with rfaₛₒₙₙₑᵢ clones pSSVI232 to pSSVI234 and pSSVI249 to pSSVI251. All six plasmids were conjugated into S. typhi TY21a and V. cholerae CVD103-HgR, and their expression was studied like for rfa_{R1} clones. Whereas expression in E. coli and S. typhi was similar, only two (pSSVI249 and 250) out of the six clones were positive in V. cholerae. To determine whether this lack of expression stemmed from incomplete information or from plasmid instability, plasmid minipreps were made from CVD103-HgR and TY21a transconjugants and used to transform back E. coli K-12 strain DH5a. Results showed that only two out of five DNAs extracted from V. cholerae had deletions. Since, however, two of the unresponding DNAs were intact and expressed the R1-type LPS after back-transfer into DH5a, we conclude that these clones lack some essential determinant for the expression of the R1 LPS core in V. cholerae. Altogether, the best clone was pSSVI250 which was positive in all strains and remained structurally unaltered, even in V. cholerae. Plasmid pSSVI249 was unstable in CVD103-HgR.

### Example 7

### Co-expression studies of S. sonnei rfb/rfc genes and E. coli R1 rfa genes in heterologous genetic backgrounds

As discussed in Example 5, the expression of S. sonnei rfb/rfc genes in S. typhi and V. cholerae candidate vaccine strains, resulted in the production of O-antigen molecules present at the cell surface in a form unbound to the LPS core. In order to test the co-expression of S. sonnei rfb/rfc genes and rfa genes encoding R1-type LPS (either rfa_{R1} or rfaₛₒₙₙₑᵢ₎, we made use of S. sonnei rfb/rfc subclones in plasmid vectors compatible with the pLAFR-5 replicon. The reactivity of double recombinant strains with both Sh5S and SH9R MAbs was examined in immunodot blots (whole cells) and immunoblots of LPS minipreps.

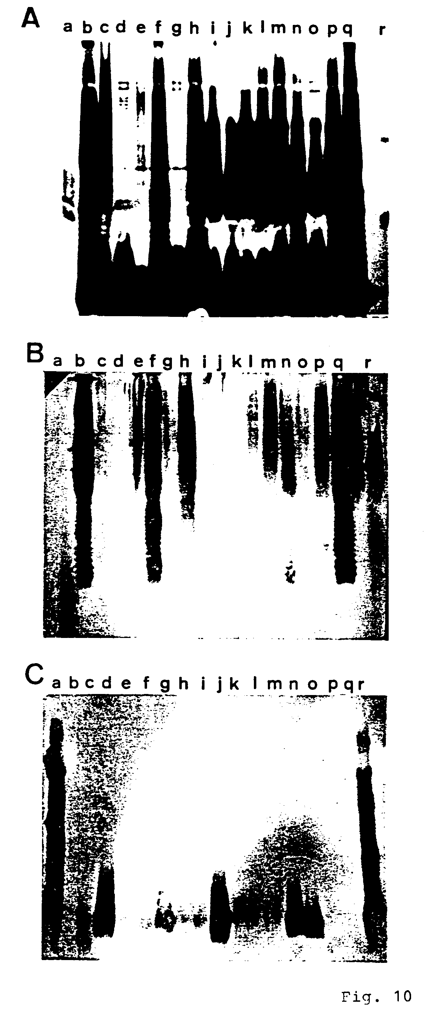

Typical results are presented in Fig. 10. In S. typhi TY21a, ladder of hmw LPS bearing S. sonnei O-antigen were observed in both silver stained SDS-PAGE gels and in an immunoblot using MAb SH5S for, e.g., the rfb/rfc clone pSSVI165 associated with the rfa_{R1} clone pSSVI123 and the rfaₛₒₙₙₑᵢ clone pSSVI250 (Fig 10, lanes f and h). In V. cholerae strain CVD103-HgR, attractive combinations were, e.g., the rfb/rfc clone pSSVI165 together with the rfa_{R1} clones pSSVI123 and pSSVI128 (lanes m and n), a stronger response being generally observed with the latter. pSSVI125 associated with pSSVI162 provided the same pattern as pSSVI128 (data not shown). A ladder of hmw LPS responding with Sh5S was also detected with rfaₛₒₙₙₑᵢ clone pSSVI250 (lane p). In all cases the presence of rfa clones directed the synthesis of LPS detectable with the R1 core-specific MAb SH9R. However, the response to Sh9R was considerably reduced for those strains carrying both plasmids together (lanes f, h, m, n, and p). This effect most likely resulted from the masking of Sh9R epitope by covalently bound O-antigen molecules.

In conclusion, it appears that the co-expression of both suitable rfa genetic determinants encoding R1-type LPS and the S. sonnei rfb/rfc locus in the candidate recombinant strains TY21a and CVD103-HgR leads to the synthesis of complete O-antigen-bearing LPS molecules of the same type as the ones present in virulent strains of S. sonnei.

### Example 8

### Immunofluorescence detection of S. sonnei O-antigen and R1-type LPS at the surface of recombinant strains

In case the S. sonnei O-antigen and/or the R1-type LPS produced in recombinant strains bearing the cloned rfb/rfc and rfa genetic determinants are exposed on the surface of the carrier cells, one should be able to detect them by immunofluorescence microscopy using the respective MAb Sh5S and Sh9R as the first antibodies. Such an analysis was performed as described (Lang et al., Hum. Antibod. Hybridomas **1:**96-103, 1990) with selected strains and clones. Data are summarized in Table 6. Recombinant strains bearing the various S. sonnei rfb/rfc clones were strongly positive when probed with MAb SH5S. In each case, the reactive material was found associated with the cell surface. This was even true for S. typhi TY21a bearing the sole rfb/rfc clones, in which the O-antigen produced is not bound to the LPS core (see Example 5). Interestingly, S. typhi TY21a containing both rfa_{R1} clone pSSVI123 and rfb/rfc clone pSSVI165 were as expected strongly positive with SH5S but only weakly positive with the core-specific MAb SH9R. The same was true for S. sonnei strain 53GII bearing clone pSSVI109. In agreement with immunoblot data (see Example 7), these observations suggest that the covalently bound O-antigen sterically prevent the access of the core-specific MAb to its target.

Thus, LPS components produced in recombinant strains under the control of the cloned rfb/rfc and rfa genetic determinants are present at the surface of the cells in a form accessible to exogeneous antibodies. This configuration may be most favourable for the stimulation of an immune response towards S. sonnei O-antigen.

### Example 9

### Immunogenicity of V. cholerae strain CVD103-HgR bearing S. sonnei rfb/rfc clone pSSVI109

In order to test if recombinant strains expressing S. sonnei O-antigen may stimulate an immune response against S. sonnei Phase I cells, the immunogenicity of V. cholerae strain CVD103-HgR and a rifampicin-resistant (Rif^{R}) derivative thereof, both bearing S. sonnei rfb/rfc clone pSSVI109 were examined via repeated intramuscular immunization of rabbits. For some unknown reason, pSSVI109 proved to be more stable in the Rif^{R} mutant strain. Control strains were CVD103-HgR and S. sonnei Phase I strain 482-79 (pWR105). Rabbits were injected three times at about 15-day intervals with a 1 ml suspension of heat-killed cells (day 0, 3-4x10⁸ cells; days 15 and 29, 10⁹ cells). At days 0, 14 and 35, blood probes were withdrawn and examined for the presence of anti-sonnei Phase I and anti-cholerae classical Inaba antibodies by agglutination and/or ELISA tests.

As shown in Table 7, rabbits immunized with the recombinant strains provided significative antibody titers against S. sonnei Phase I and V. cholerae cells as determined by agglutination tests and by ELISA assays using S. sonnei phase I and V. cholerae purified LPS as the coating antigen.

Thus, it appears that S. sonnei O-antigen molecules produced by recombinant V. cholerae live vaccine strains expressing the cloned S. sonnei rfb/rfc genetic determinant are accessible to the immune system of vaccinated rabbits and stimulate a significant production of serum antibodies against both V. cholerae and S. sonnei Phase I LPS molecules.

### Example 10

### Cloning of S. typhi genes involved in the production of H₂S

Unlike the wild type strain TY2, S. typhi strain TY21a is known to have a H₂S⁻ phenotype (white instead of black colonies on Kliger-Iron-Agar, KIA, plates) (Silva et al., J. Infect. Dis. **155:**1077-1078, 1987). Thus, the genes responsible for the production of H₂S on KIA plates may be a good target for chromosomal integration of cloned genes into TY21a. Accordingly, we isolated the corresponding genetic locus (H₂S locus) from the parent strain TY2.

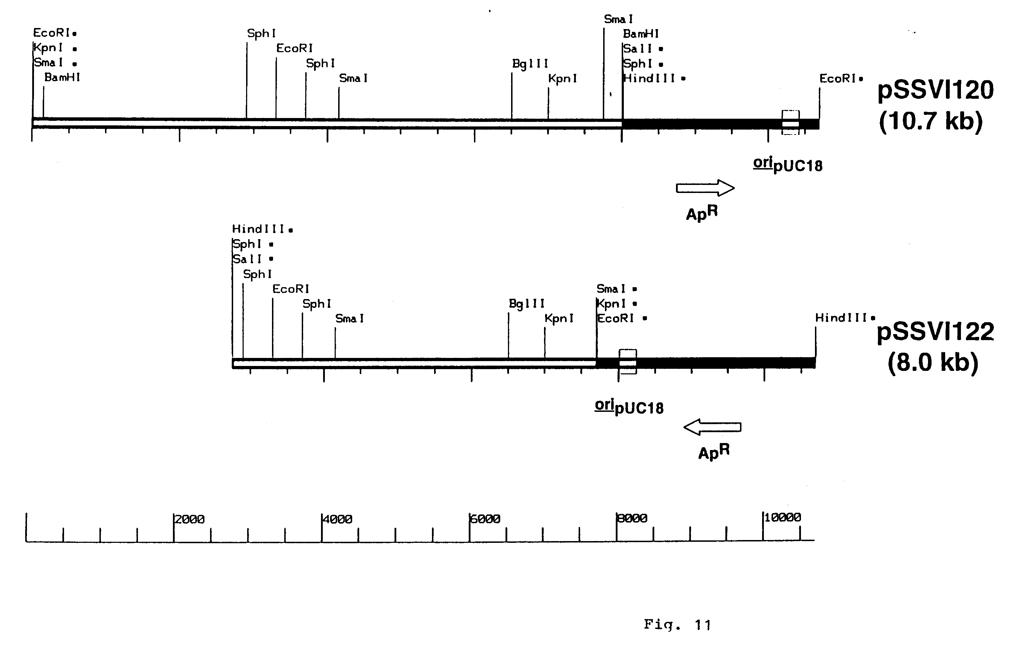

The H₂S locus was cloned from a partial Sau3AI chromosomal gene bank of S. typhi strain TY2 established in the BamHI site of vector pUC18. E. coli K-12 DH5a competent cells were transformed with the bank and plated onto KIA plates containing 100 mg/ml of ampicillin. Three stable clones giving rise to black colonies were isolated and named pSSVI120, 121, and 122. Restriction mapping showed that the cloned DNAs partially overlap. Restriction maps of clones pSSVI120 and 122 are shown in (Fig. 11).

Plasmids pSSVI120, 121, and 122 were introduced into TY21a via electroporation. However, none of the transformants presented black colonies on KIA plates. In parallel we observed that TY21a is transformed at a low frequency with high copy number vectors, such as pUC18, as compared with low copy number vectors based on the pSC101 replicon, such as pGB2. Therefore, we subcloned the H₂S locus on a 4.5 kb EcoRI fragment from plasmid pSSVI122 into the EcoRI site of plasmid pGB2 to give pSSVI132 and 133, carrying the 4.5 kb insert in either orientation. Both plasmids still conferred the expected phenotype in DH5a while being negative in TY21a. However, since plasmid DNA extracted from one TY21a transformant still conferred the H₂S⁺ phenotype when transferred back into DH5a, one may conclude that the cloned genes alone are unable to complement TY21a deficiency. This suggests that the H₂S⁻ phenotype of TY21a results of mutation(s) in either another or in more than one region of the chromosome.

### Example 11

### Design of vectors for targeted integration of cloned genes into the S. typhi H₂S chromosomal locus

The integration of large (e.g. 20-30 kb) DNA fragments into the chromosome of TY21a may require the presence on the integration vector of an extended DNA region of sequence similarity with the target locus. In order to derive an integration cassette, a 6.8 kb KpnI-BamHI fragment from pSSVI120 (Fig. 11) was subcloned into vector pMTL21par/NE, giving plasmid pSSVI148. A 24 bp synthetic oligonucleotide corresponding to BamHI, NotI, and SalI restriction sites (BNS cassette) was then introduced into either the EcoRI or the SmaI sites approximately in the middle of pSSVI120 insert, resulting in plasmids pSSVI150 and pSSVI151, respectively.

In order to force integration into the chromosome, the genes to be integrated have to be introduced into the recipient strain on a so-called suicide vector based, e.g., on the temperature-sensitive plasmid pMAK700. The suicide property of plasmid pMAK700 comes out of the fact that it is replication deficient at high temperature (42 to 44°C) whereas it normally replicates at the permissive temperature (30°C) (Hamilton et al. J. Bacteriol. **171:**4617-4622, 1989). Therefore, rare integration events into the chromosome of the recipient strain may be selected for by shifting the cell population from the permissive to the non-permissive temperature and selecting for the plasmid-encoded chloramphenicol resistance (Cm^{R}) marker. Later on, the specific re-excision of the plasmid out of the chromosome leads to chloramphenicol-sensitive (Cm^{R}) plasmid-free cells (Hamilton et al. J. Bacteriol. **171:**4617-4622, 1989). In order to facilitate the introduction of cloned genes into candidate vaccine strains, pMAK700 was first modified by the introduction, into its blunted BamHI site, of a blunted EcoRI-BamHI fragment of about 0.75 kb from plasmid pJFF350 bearing oriT, a specific DNA sequence allowing for the conjugational transfer from donor strains, such as E. coli K-12 S17.1, via the RP4/RK2 conjugation system (Fellay et al., Gene **76:**215-226, 1989; Simon et al., Bio/technology **1:**784-791). This gave rise to plasmid pMAK700oriT.

Another suicide vector may be plasmid pGP704 (Miller and Mekalanos, J. Bacteriol., **170:**2575-2583, 1988). pGP704 bears the plasmid R6K replication origin without the genetic determinant (pir gene) for the p effector protein indispensable to its replication. Its suicide behavior results from the fact that replication is only made possible in permissive E. coli K-12 host strains (e.g. SM10lpir or SY327lpir) carrying the pir gene integrated in the chromosome. pGP704 is also mobilizable from strain SM10lpir via the RP4/RK2 conjugation system (Miller and Mekalanos, J. Bacteriol., **170:**2575-2583, 1988). pGP704 contains three BamHI restriction sites. However, since the presence of a unique BamHI site may later be useful for the cloning of, e.g., the S. sonnei rfb/rfc locus into the integration cassette, the BamHI sites of pGP704 were eliminated via enzymatic treatment. The resulting vector was named pGP704.2.

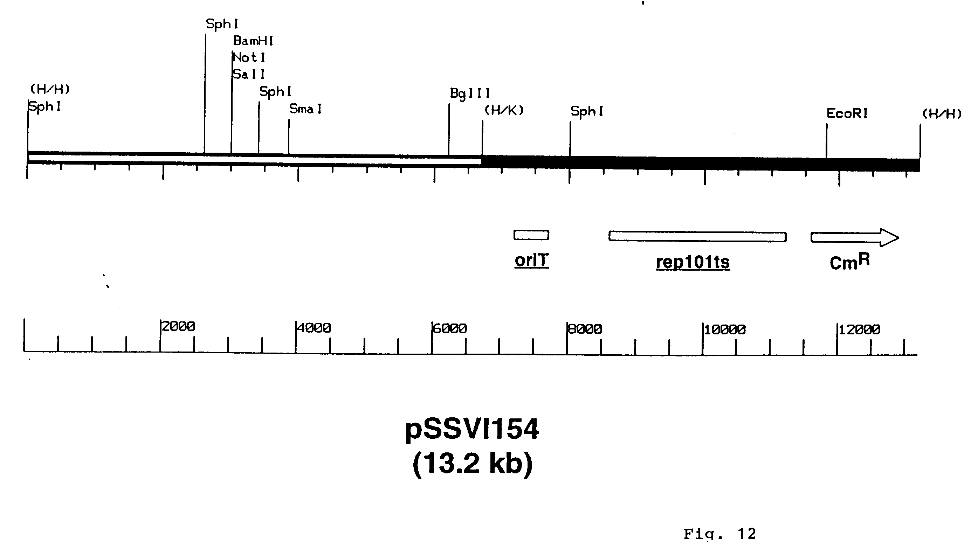

Two integration vectors bearing H₂S integration cassettes were constructed (Fig. 12). In the first one, pSSVI154 (about 13 kb), the blunted KpnI-HindIII insert of about 7 kb from pSSVI150 was cloned into the blunted HindIII site of vector pMAK700oriT. In the second integration vector, pSSVI160 (about 7.4 kb), the blunted HindIII-SmaI insert of about 4 kb from pSSVI150 was cloned into the SmaI site of vector pGP704.2.

Subcloning of the S. sonnei rfb/rfc locus is possible from, e.g., clone pSSVI165 into the BNS restriction cassette present in the integration vectors pSSVI154 and pSSVI160. The resulting integration clones may then be transferred into S. typhi TY21a for chromosomal integration of the S. sonnei rfb/rfc locus.

### Example 12

### Characterization of TY21a ilv character and cloning of the S. typhi ilv genetic locus

The auxotrophic requirements of S. typhi TY21a were first examined by inoculating the strain into a minimal salt medium supplemented with different amino acids at the concentration of 20 mg/ml. A strict requirement was observed for L-isoleucine and L-valine which had to be supplemented both together. Such a phenotype indicates that TY21a bears a mutation in the ilv genetic locus encoding enzymes involved in the biosynthesis of both amino acids. Other essential amino acids were L-cysteine and L-methionine, either one promoting growth when added alone to the minimal medium containing L-isoleucine and L-valine. Such a phenotype is compatible with a genetic defect in a metabolic step for which these amino acids may be needed (e.g. as donors of a sulfur atom). Finally, L-tryptophane slightly stimulated the growth of TY21a but was dispensable.

The nature of the ilv mutation carried by TY21a was determined by cross-feeding on minimal plates using defined ilv mutants of E. coli K-12. TY21a was fed by ilvE, but not by ilvA, ilvB, ilvG, ilvM, ilvC, or ilvD mutants. In turn, TY21a was able to cross-feed ilvA (weakly), ilvB, ilvG, ilvM, and ilvC, but not ilvD or ilvE E. coli K-12 mutants. These results indicate that TY21a is mutated in the ilvD gene encoding dihydroxyacid dehydrase. In both E. coli K-12 and S. typhimurium, the ilvD gene belongs to an operon structure (ilvGMEDA operon). It is therefore likely that the genetic defect of TY21a maps in the corresponding ilvGMEDA operon of S. typhi.

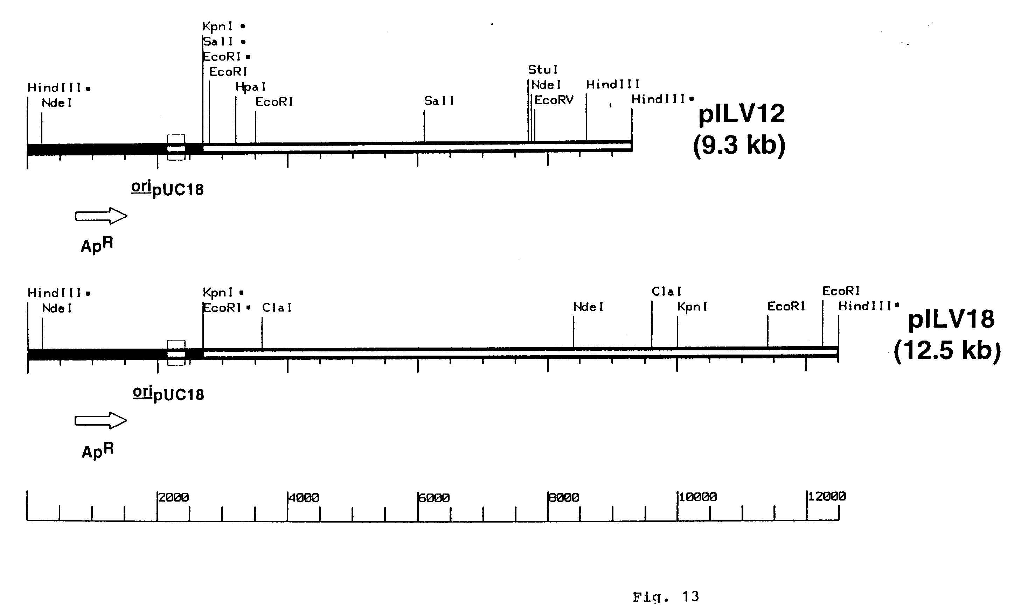

In order to design a cassette for chromosomal integration into TY21a, the corresponding ilv locus was cloned from a gene of the chromosomal gene bank of S. typhi TY2 described in Example 9 by complementation of the E. coli K-12 ilvC mutant strain AB1419. The ilvC locus is adjacent to the ilvGMEDA operon in the E. coli K-12 chromosome so that some isolated clones complementing an ilvC mutation may extend into the neighbouring region encompassing the ilvGMEDA operon. Two complementing plasmid clones, named pILV12 and pILV18, were analyzed with restriction enzymes and shown to bear inserts providing both a sufficient size (6.6 and 9.7 kb, respectively) for the chromosomal integration of large pieces of DNA, and unique restriction sites located roughly in the middle of the inserts, suitable for the cloning of, e.g., the S. sonnei rfb/rfc locus (Fig. 13). It is note-worthy that pILV12, but not pILV18, also complemented the ilv defect of both the E. coli K-12 ilvD mutant strain AB1280 and S. typhi TY21a. This shows that clone pILV12, but not pILV18, most likely includes both the ilvGMEDA operon and the adjacent ilvC locus. Herein, the insert present in pILV12 will be referred to as the S. typhi ilv genetic locus.

### Example 13

### Design of a vector for targeted integration of cloned genes into the S. typhi ilv chromosomal locus

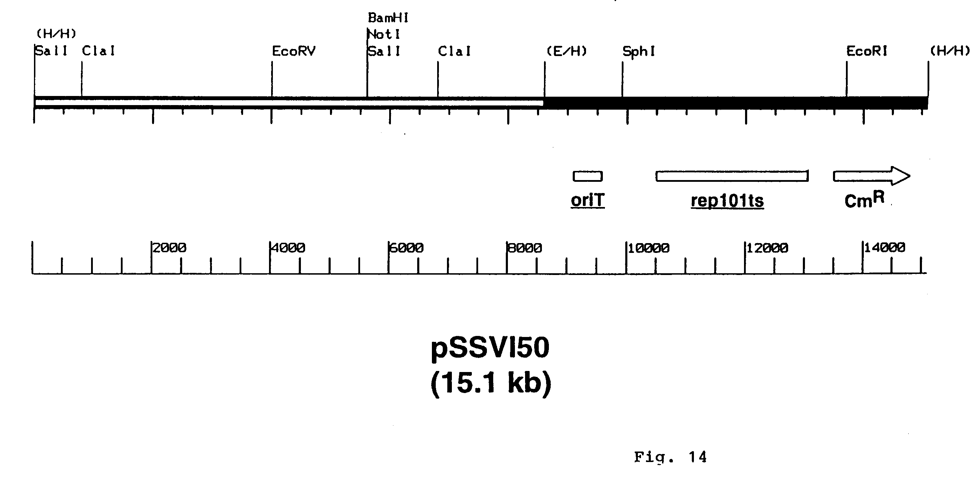

In order to derive a vector for chromosomal integration into the ilv genetic locus, the SalI-HpaI fragment of about 6 kb from pILV12 was blunted and subcloned into the blunted HindIII site of vector pMAK700oriT (see Example 10), giving rise to plasmid pILV23. The unique BamHI site present in the insert of pILV23 was then enzymatically removed, resulting in pILV24. Finally, the BNS restriction cassette (see Example 10) was introduced into the unique KpnI site present in the middle of the insert, giving the integration vector pSSVI50 (Fig. 14).

Subcloning of, e.g., the S. sonnei rfb/rfc locus is possible into the BNS restriction cassette of vector pSSVI50. The resulting integration clones may then be introduced into S. typhi TY21a for chromosomal integration of the S. sonnei rfb/rfc locus.

### Example 14

### Design of vectors for the integration of cloned genes into the V. cholerae hlyA chromosomal locus

An attractive target for chromosomal integration of foreign genes into the chromosome of V. cholerae live vaccine strains is the hlyA locus, encoding the hemolysin virulence determinant. From plasmid pJMK10, a suitable integration cassette of about 10 kb (hlyA/mer integration cassette), encompassing the hlyA locus disrupted with a mercury resistance (mer) determinant, was designed and subcloned into both suicide vectors pMAK700oriT and pGP704.2.

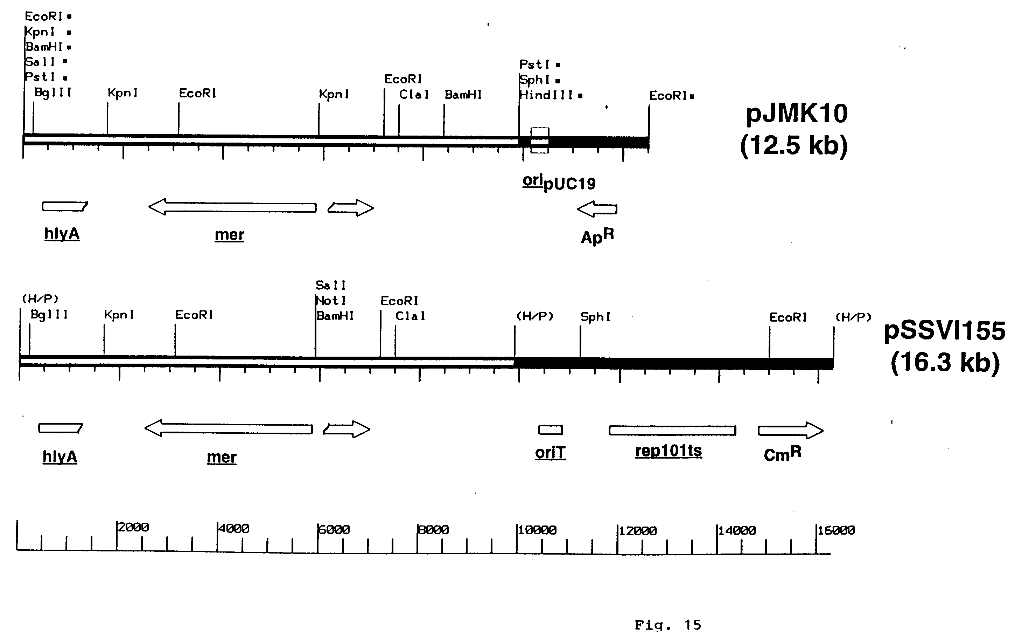

Based on pMAk700oriT, two integration vectors were derived, pSSVI155.1 and pSSVI155.2, carrying the hlyA/mer cassette as a blunted PstI fragment in either orientation within the blunted HindIII site of pMAk700oriT (Fig. 15). Such plasmids bear resistance determinants to both chloramphenicol (Cm^{R}) and HgCl₂.

Two integration vectors were derived making use of pGP704.2. The first one, pSSVI159, consists in plasmid pGP704.2 in which the 0.7 kb PstI fragment, including most of pGP704.2 ampicillin resistance (Ap^{R}) determinant, was replaced by the 10 kb PstI hlyA/mer integration cassette. Consequently, pSSVI159 encodes resistance to HgCl₂ only. A second integration vector was constructed via cloning of the hlyA/mer cassette into pGP704.2 partially digested with PstI, giving plasmid pSSVI164. The latter integration vector carries the 10 kb PstI cassette in the PstI site of pGP704.2 polylinker and encodes resistance to both ampicillin and HgCl₂.

Subcloning of, e.g., the S. sonnei rfb/rfc locus is allowed into the BNS restriction cassette present in plasmids pSSVI155.1, pSSVI155.2, pSSVI159, and pSSVI164, and the resulting integration clones may then be introduced into, e.g, V. cholerae CVD103-HgR for chromosomal integration of the S. sonnei rfb/rfc locus. It is noteworthy that the chromosomal integration of foreign genetic determinants using the hlyA/mer integration cassette described here is accompanied by the integration of an active mer genetic determinant allowing for the later identification of the vaccine strain.

### Example 15

### Integration of the S. sonnei rfb/rfc locus into the chromosome of V. cholerae strain CVD103-HgR

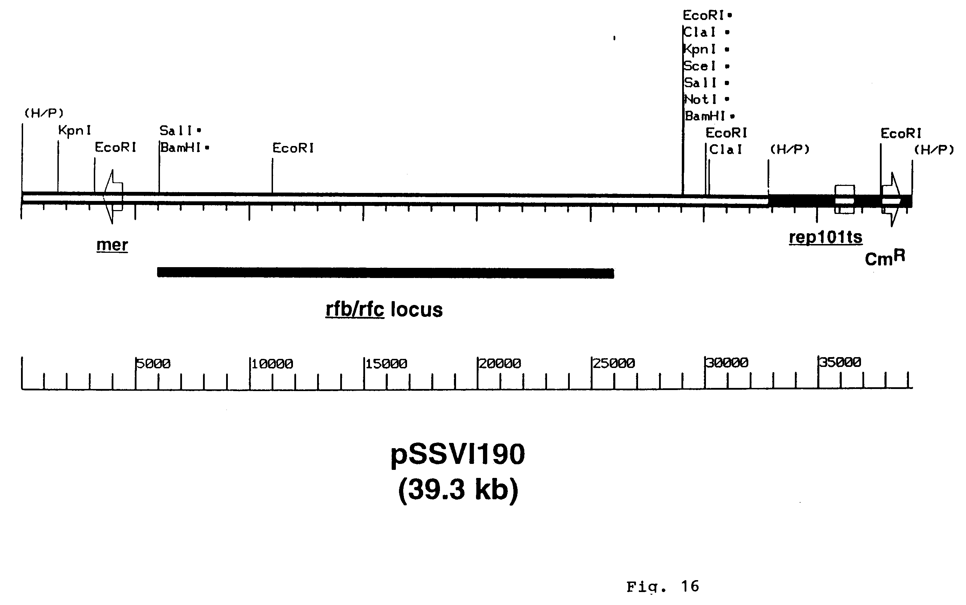

The 23 kb insert of S. sonnei rfb/rfc clone pSSVI165 (Fig. 3) was modified so that it is bordered with SalI restriction sites. The ensuing 23 kb SalI fragment was then subcloned into the unique SalI site of integration vector pSSVI155.1, giving pSSVI190 (Fig. 16). The latter clone stably expressed S. sonnei O-antigen in E. coli K-12 when propagated at the permissive temperature (30°C). Plasmid pSSVI190 was then transferred via conjugation/mobilization from E. coli K-12 strain S17.1 into a spontaneous rifampicin-resistant (Rif^{R}) derivative of V. cholerae CVD103-HgR. The resulting strain CVD103-HgR Rif^{R} (pSSVI190) expressed high amounts of S. sonnei O-antigen when grown at 30°C.

In order to select for those cells which may have integrated plasmid pSSVI190 into their chromosome, a culture of CVD103-HgR Rif^{R} (pSSVI190) growing exponentially in BHI medium at 30°C was streaked onto BHI plates with or without chloramphenicol (17 mg/ml). Incubation was in parallel at 30°C and 42°C. Incubation of selective plates at the non-permissive temperature resulted in a survival of about 5.10⁻⁵ relative to the same plates incubated at 30°C or to nonselective plates incubated at 42°C. Some rare colonies from selective plates at 42°C were shown to agglutinate in the presence of both S. sonnei Phase I-specific and V. cholerae Inaba-specific antisera. Agglutinating colonies were further streaked onto selective plates and incubated at 42°C. Newly grown colonies still agglutinated in the presence of both antisera and presented a strong reaction with MAb Sh5S, indicating that they stably expressed S. sonnei O-antigen. Streaking of the colonies on non-selective plates and incubation at 30°C, followed by re-streaking on non-selective plates at 42°C resulted in the isolation of chloramphenicol-sensitive colonies which still strongly reacted with S. sonnei-specific antiserum and MAb SH5S. Such colonies when further streaked on non-selective (BHI) plates only gave rise to positive O-antigen producing colonies at both 30°C and 42°C. Examination of the DNA content of such strains failed to reveal plasmid DNA whereas strain CVD103-HgR Rif^{R} (pSSVI190) grown at 30°C presented the expected plasmid pattern.

From the above data we conclude that colonies isolated on selective plates at 42°C carry plasmid pSSVI190 in a form integrated in the chromosome. Later growth on non-selective plates at 30°C followed by 42°C resulted in the isolation of plasmid-free strains via excision of the plasmid replicon from the chromosome and later curing of the cytoplasmic plasmid form. The resulting strains bear S. sonnei rfb/rfc locus integrated in their chromosome. One of those strains was named CH2 (Table 4). Southern hybridization analysis of CH2 chromosomal DNA using specific probes confirmed (i) that the S. sonnei rfb/rfc locus is integrated in the hlyA locus in the expected orientation, and (ii) that the strain is devoid of plasmid vector sequence.

Strain CH2 proved to be extremely stable with respect to the expression of S. sonnei O-antigen. Indeed, after 45 generations of growth in BHI medium at 30°C and streaking on BHI plates, 100% of the colonies were positive in an immunoblot with MAb Sh5S. Finally, CH2 presented the same HgCl₂ resistance level as CVD103-HgR (overnight growth at 30°C in liquid BHI medium containing 60mM HgCl₂, versus 30 to 40 mM HgCl₂ for CVD103).

### TABLES

Table 1
   Bacterial strains used in the characterization of MABs
Table 2
   Binding properties of anti-S. sonnei LPS MABs
Table 3
   Binding activity of MAb SH9R to LPS isolated from clinically relevant strains of E. coli and P. aeruginosa
Table 4
   Bacterial strains and plasmids used in the cloning and expression studies of the S. sonnei rfb/rfc genes and the rfa genetic determinants encoding R1-type LPS molecules
Table 5
   Expression studies of E. coli R1 rfa genes in heterologous genetic backgrounds
Table 6
   Immunofluorescence microscope analysis of recombinant strains expressing S. sonnei O-antigen and/or R1-type LPS
Table 7
   Test of the immunogenicity of the V. cholerae recombinant vaccine strain CVD103-HgR expressing S. sonnei O-antigen

**Table 1**

| **Bacterial strains used in the characterization of monoclonal antibodies.** | | | | |
|---|---|---|---|---|
| Strain | O-type | LPS structure | Expected core chemotype | Origin and comments |
| **Shigella** | | | | |
| son. 53GI | D | Form I | R1 | Phase I (1)^{a} |
| son. 53GII | / | Form II | R1 | Phase II variant of 53GI (this study) |
| son.482-79(pWR105) | D | Form I | R1 | WR6050, Ph. Sansonetti (2) |
| son. PCM1985 | / | rough | R1 | E. Romanowska |
| boyd. 1 | C1 | smooth | u | type 1, ATCC 9207 |
| boyd. 3 | C3 | smooth | R1 | type 3, ATCC 8702 |
| dys. 1 | A1 | smooth | R4 | type 1, ATCC 9361 |
| flex. 4b | B4b | smooth | R3 | type 4b, ATCC 12024 |
| flex. 6 | B6 | smooth | R1 | type 6, ATCC 12025 |
| flex. S323 | B6 | smooth | R1 | type 6, E. Romanowska (3) |
| flex. R6 551 | / | rough | R1 | deep rough mutant of flex. type 6 E. Romanowska (4) |
| flex. R6 288 | / | rough | R1 | same origin as R6 551 |

| **P. shigelloides** | | | | |
|---|---|---|---|---|
| M51 | son. | smooth | R1? | (Bader) ATCC 14029 |
| C27 | son. | smooth | R1? | (Ferguson) ATCC 14030 |

| **E. coli** | | | | |
|---|---|---|---|---|
| HB101 | / | rough | K-12 | (5) |
| HF4704 | / | rough | R1 | A.A Lindberg (6) |
| O8:K8(L):H4 | O8 | smooth | R1/R2? | ATCC 23504 |
| O9:K9(B):H12 | O9 | smooth | R1 | ATCC 23505 |
| O14:K7(L):NM | O14 | smooth | R4 | ATCC 19110 |
| O100:H509a | O100 | smooth | R2 | NCTC 9100 |

| **S**. **typhi** | | | | |
|---|---|---|---|---|
| TY21a | D1(9,12) | s-rough | Ra | galE (7) |
| TY37(pWR105) | D1(9,12) | s-rough | Ra | Rifampicin-resistant derivative of TY21a bearing plasmid from 482-79(pWR105) (this study) |

| **S. typhimurium** | | | | |
|---|---|---|---|---|
| TV119 | / | rough | Ra | K.E. Sanderson |
| son., sonnei; boyd., boydii; flex., flexneri; u, unknown; s-rough, semi-rough phenotype linked to the galE genetic background. | | | | |

| | | | | |
|---|---|---|---|---|
| **a) References are:** (1) Kopecko et al., Infect. Immun. **29:**207-214, 1980. (2) Sansonetti et al. Infect. Immun. **34:**75-83, 1981. (3) Przondo-Hessek and Romanowska, Acta Microbiol. Pol. **27:**97-102, 1978. (4) Katzenellenbogen and Romanowska, Eur. J. Biochem. **116:**205-211, 1980. (5) Boyer and Roulland-Dussoix, J. Mol. Biol. **41:**459-472, 1969. (6) Jansson et al., Eur. J. Biochem. **115:**571-577, 1981. (7) Germanier and Fürer, J. Infect. Dis. **131:**553-558, 1975. | | | | |

**Table 2**

| **Binding properties of anti-S. sonnei LPS monoclonal antibodies.** | | | | | | |
|---|---|---|---|---|---|---|
| MAb | Isotype^{a} | titer^{b} | | Immunoblot pattern^{c} | | |
| | | S.s.I | S.s.II | S.s.I | | S.s.II |
| | | | | O-Ag | core | core |
| Sh5S | IgM | >128 | <2 | + | - | - |
| Sh11S | IgM | >128 | <2 | + | - | - |
| Sh9R | IgM | 16 | 64 | - | + | + |

| | | | | | | |
|---|---|---|---|---|---|---|
| (a) Isotype determination by ELISA as described in the test. | | | | | | |
| (b) Antibody titers were determined by ELISA in wells coated with purified S. sonnei Phase I (S.s.I) or Phase II (S.s.II) LPS. Titers are expressed as the reciprocal value of the dilution resulting in an OD of 0.4. | | | | | | |
| (c) Binding to high molecular weight O-antigen-bearing (O-Ag) or low molecular weight (core) LPS components. | | | | | | |

**Table 3**

| **Binding activity of Sh9R MAb to LPS isolated from clinically relevant strains of E. coli and P. aeruginosa.** | | |
|---|---|---|
| Bacterial species | O-serotype | Binding activity^{a} |
| E. coli | O1 | + |
| | O2 | + |
| | O4 | - |
| | O6 | + |
| | O7 | + |
| | O12 | - |
| | O15 | - |
| | O18 | + |
| | O25 | + |
| | O75 | + |
| P. aeruginosa | O1^{b} | - |
| | O2 | - |
| | O3 | - |
| | O4 | - |
| | O6 | - |
| | O7 | - |
| | O10 | - |
| | O11 | - |
| | O16 | - |

| | | |
|---|---|---|
| (a) Determined by immunoblot using purified LPS as the antigen. (+), MAb reacts with the core-lipid A region; (-), no reaction. | | |
| (b) According to the International Antigenic Typing System (IATS) | | |

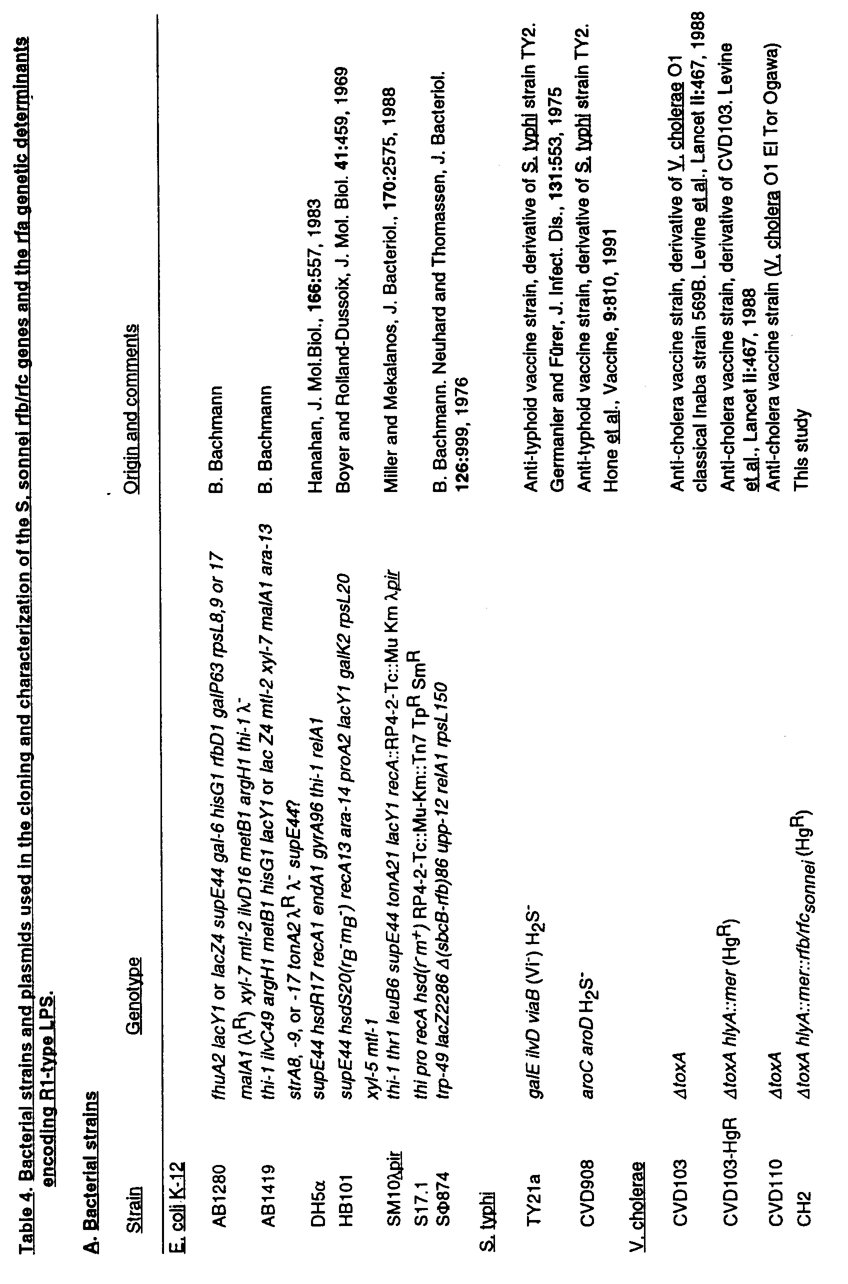

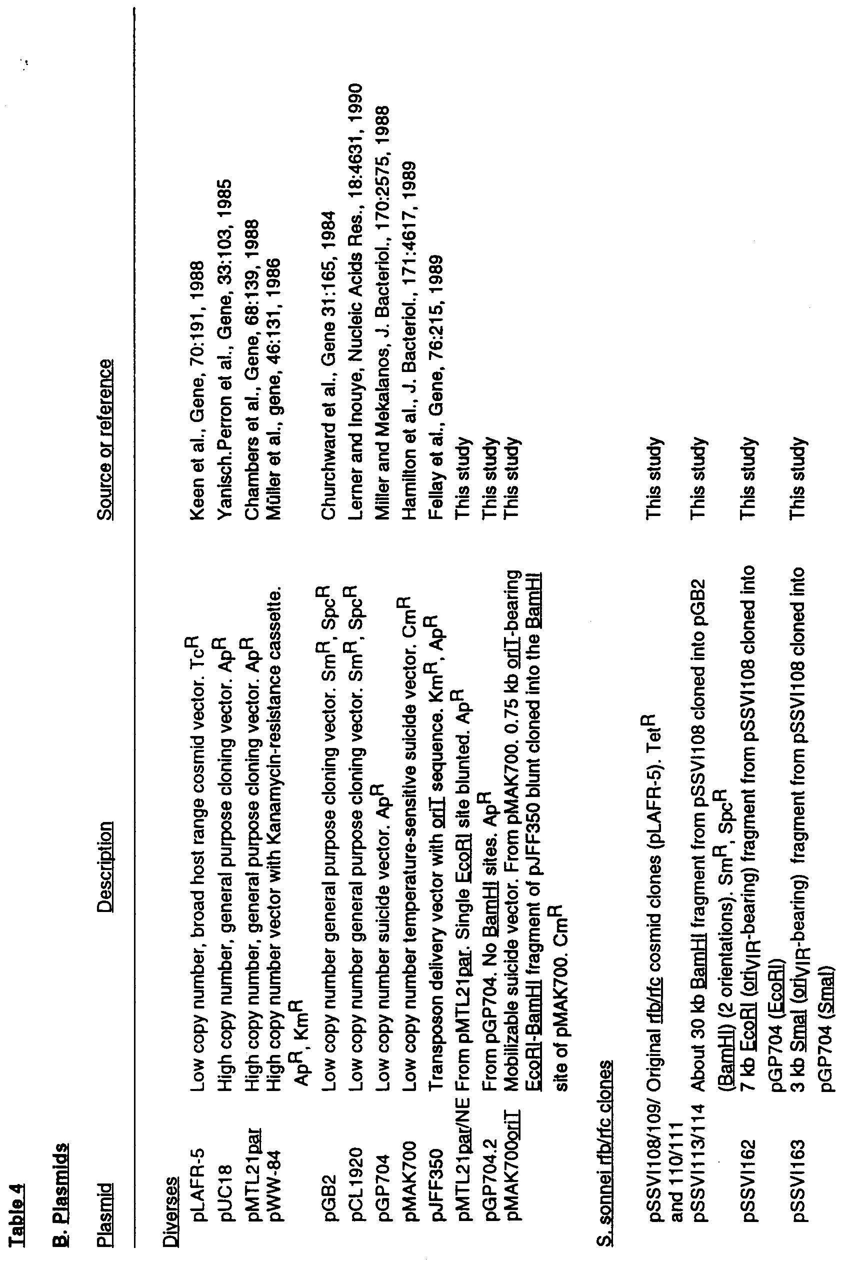

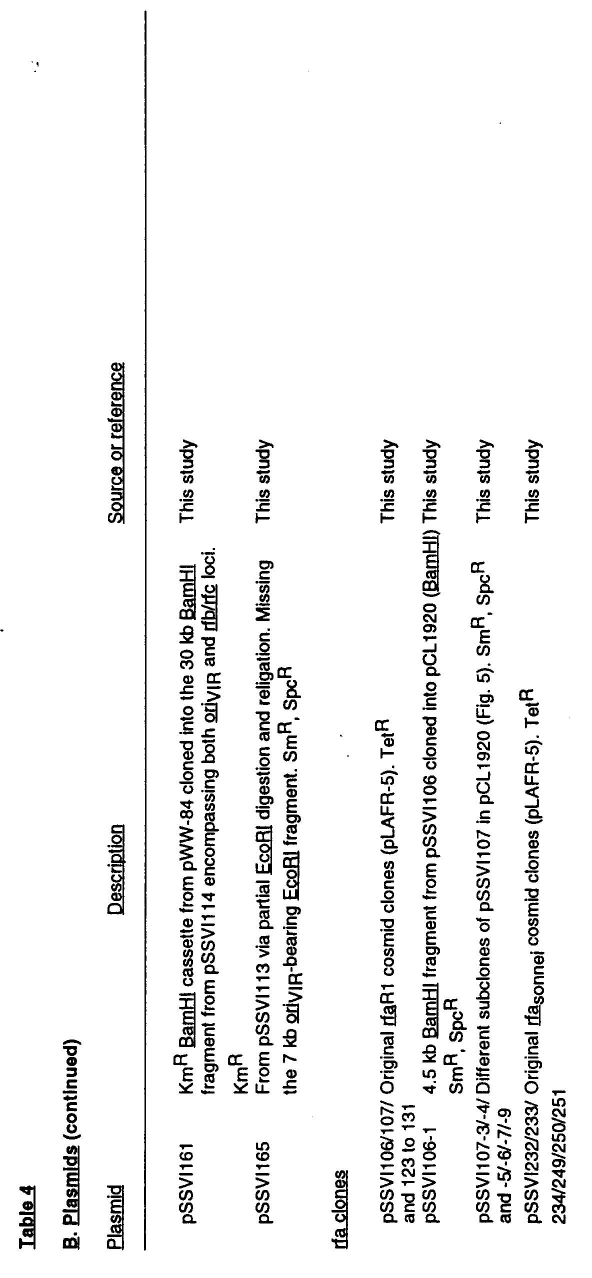

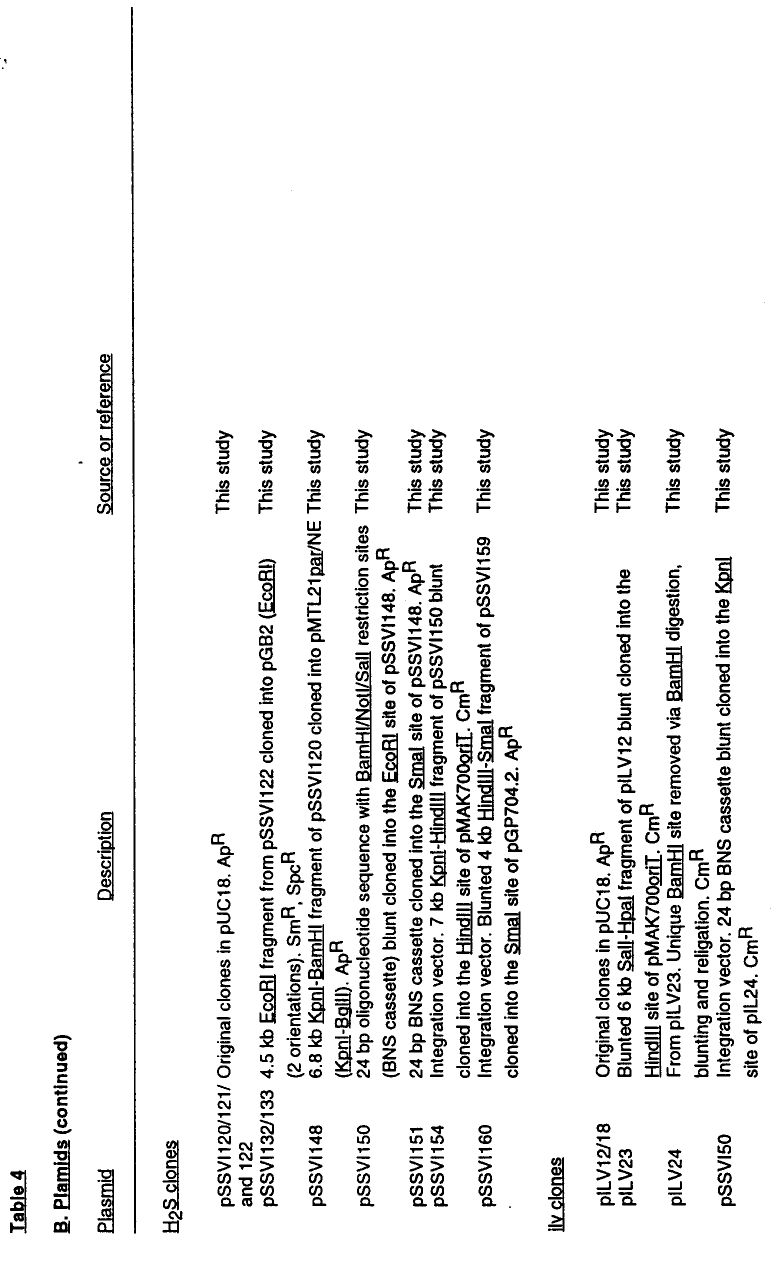

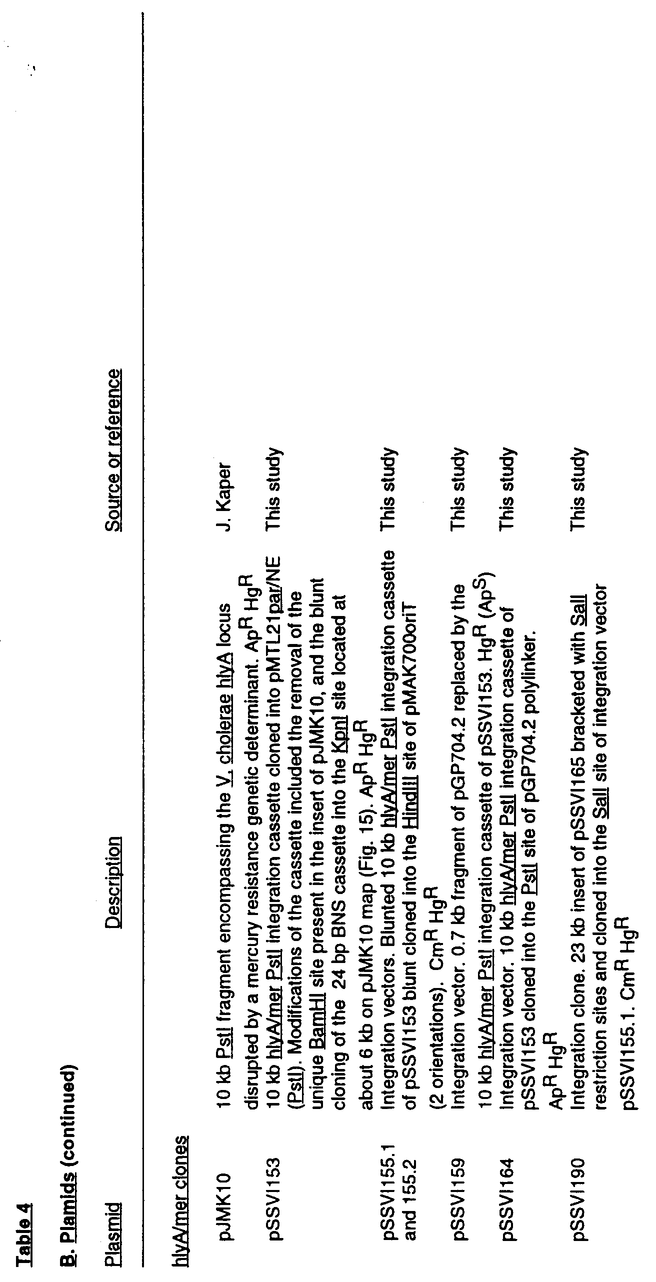

**Table 5**

| **Expression studies of E. coli R1 rfa genes in heterologous genetic backgrounds.** | | | |
|---|---|---|---|
| Strain | pSSVI plasmid | Detection of R1-type LPS with MAb Sh9R | |
| | | Whole cells immunodots | LPS immunoblots |
| E. coli K-12 | | | |
| | no | - | - |
| | 106/107/123 | +++ | +++ |
| | 124 to 130/107-5/107-6 | +++ | n.t. |
| | 107-3/107-4 | ++ | n.t. |
| S. typhi TY21a | | | |
| | no | - | - |
| | 106/107/123 | +++ | +++ |
| | 124 to 126/128/107-5 | +++ | n.t. |
| | 127/130 | + | n.t. |
| | 107-3 | - | n.t. |
| V. choleraeCVD103-HgR | | | |
| | 123/124/125/128 | +++ | +++ |
| | 106 | ++ | + |
| | 107^{a} | + | + |
| | 126/127/130/107-5 | - | n.t. |

| | | | |
|---|---|---|---|
| a) clone pSSVI107 was highly unstable in CVD103-HgR. | | | |
| +++, strong reaction; ++, intermediate reaction; +, weak reaction; -, no reaction; n.t., not tested; | | | |

**Table 6**

| **Immunofluorescence microscope analysis of recombinant strains expressing S. sonnei O-antigen and /or R1-type LPS.** | | | |
|---|---|---|---|
| Strain | pSSVI plasmid(s) | First antibody | |
| | | MAb Sh5S | MAb Sh9R |
| E. coli K-12 | | | |
| DH5α | no | - | n.t. |
| HB101 | 109 | +++ | n.t. |
| SΦ874 Δ(rfb/rfc) | no | - | n.t. |
| SΦ874 Δ(rfb/rfc) | 165 | +++ | n.t. |
| HB101 | 123 | n.t. | +++ |

| S. sonnei | | | |
|---|---|---|---|
| 53GII | no | n.t. | +++ |
| 53GII | 109 | n.t. | ++ |

| S. typhi | | | |
|---|---|---|---|
| TY21a | no | - | n.t. |
| TY21a | 109 | +++ | n.t. |
| TY21a | 123+165 | +++ | + |

| V. cholerae | | | |
|---|---|---|---|
| CVD103-HgR | no | - | - |
| CVD103-HgR | 109 | +++ | n.t. |
| +++, strong reaction; ++, intermediate reaction; +, weak reaction; n.t., not tested. | | | |

**Table 7**

| **Tests of the immunogenicity of the V. cholerae recombinant strain CVD 103-HgR expressing S. sonnei O-antigen.** **A. Agglutination tests (day 14 sera)** | | | | | | | | | |
|---|---|---|---|---|---|---|---|---|---|
| Tested strain | **Rabbits immunized with**^{**a**} | | | | | | | | |
| | S. sonnei 482-79(pWR105) | | V. cholerae CVD103-HgR | | | V. cholerae CVD103-HgR (pSSVI109) | | V. cholerae^{b} CVD103-HgR Rif^{R} (pSSVI109) | |
| | A^{c} | B | C | D | E | F | G | H | I |
| S. sonnei 482-79 (pWR105) | ++ | ++ | - | - | - | + | +/- | + | + |
| V. cholerae CVD103-HgR | - | - | ++ | ++ | ++ | ++ | +/- | +/- | ++ |

| **B. ELISA tests (day 35 sera)** | | | | | | | | | |
|---|---|---|---|---|---|---|---|---|---|
| LPS purified from | **Rabbits** | | | | | | | | |
| | A | B | C | D | E | F | G | H | I |
| S. sonnei | 9'630 | 5'750 | <100 | / | <100 | 1'680 | 4'150 | 2'670 | 3'020 |
| V.cholerae | <100 | <100 | 7'940 | 19'450 | 17'700 | 19'300 | 12'350 | 8'121 | 4'205 |

| | | | | | | | | | |
|---|---|---|---|---|---|---|---|---|---|
| a) Rabbits were immunized intramuscularly with 3-4x10⁸ (day 0), or about 10⁹ (day 15 and 29) heat-killed cells. | | | | | | | | | |
| b) Strain CVD103-HgR Rif^{R}(pSSVI109) is a rifampicin-resistant derivative of strain CVD103-HgR. | | | | | | | | | |
| c) A, B, ...,I, designate different rabbits immunized with bacterial strains as described in Table A. | | | | | | | | | |
| For agglutination tests, undiluted and non-preadsorbed 14 days sera were used. In the ELISA tests, purified LPS from either S. sonnei 53GI or V. cholerae CVD-103 were used as the coating antigen. Titers are expressed as the reciprocal value of the dilution resulting in an OD of 0.4. | | | | | | | | | |

### FIGURES

- Figure 1: Reactivity of Sh5S and Sh9R MAbs towards LPS isolated from various enterobacterial species
- Figure 2: Restriction maps of the S. sonnei rfb/rfc clones pSSVI109 and pSSVI113
- Figure 3: Restriction map of the S. sonnei rfb/rfc clone pSSVI165
- Figure 4: Restriction maps of rfa_{R1} clones pSSVI106, 107, 123, 125, and 128
- Figure 5: Restriction maps and expression levels of different subclones derived from rfa_{R1} clones pSSVI106 and pSSVI107
- Figure 6: Restriction map of the rfaₛₒₙₙₑᵢ clone pSSVI250
- Figure 7: Expression of the S. sonnei rfb/rfc clone pSSVI109 in heterologous genetic backgrounds
- Figure 8: Expression of the S. sonnei rfb/rfc locus in E. coli K-12 strain SF874 Drfb/rfc
- Figure 9: Expression of rfa clones encoding R1-type LPS in heterologous genetic backgrounds
- Figure 10: Co-expression of S. sonnei rfb/rfc locus and rfa loci encoding R1-type LPS in heterologous genetic backgrounds
- Figure 11: Restriction maps of S. typhi H₂S clones pSSVI120 and pSSVI122
- Figure 12: Restriction map of integration vectors pSSVI154 (S. typhi H₂S locus).
- Figure 13: Restriction maps of the S. typhi ilv clones pILV12 and pILV18
- Figure 14: Restriction maps of integration vector pSSVI50 (S. typhi ilv locus)
- Figure 15: Restriction maps of hlyA-mer clone pJMK10 and of integration vector pSSVI155.1 (V. cholerae hlyA locus)
- Figure 16: Restriction map of integration clone pSSVI190 (V. cholerae hlyA locus)

### FIGURE LEGENDS

### Figure 1

**Reactivity of Sh5S and Sh9R MAbs towards LPS isolated from various enterobacterial species.** Silver staining (A) of LPS minipreparations run on a 7.5-20% gradient SDS-PAGE gel and immunoblot analysis using Sh5S (B) and Sh9R (C) MAb, respectively. Lanes a and u, pre-stained low molecular weight protein standard (Bio-Rad); b, S. sonnei 53GI; c, S. sonnei 53GII; d, S. sonnei 482-79 (pWR105); e, S. dysenteriae type 1 (ATCC 9361); f, S. boydii type 1 (ATCC 9207); g, S. boydii type 3 (ATCC 8702); h, S. flexneri type 4b (ATCC 12024); i, S. flexneri type 6 (S323); j, S. flexneri type 6 (ATCC 12025); k, P. shigelloides M51 (ATCC 14029); l, P. shigelloides C27 (ATCC 14030); m, E. coli HF4704; n, E. coli O8 (ATCC 23504); o, E. coli O9 (ATCC 23505); p, E. coli O14 (ATCC 19110); q, E. coli O100 (NCTC 9100); r, E. coli HB101; s, S. typhi TY21a; t, S. typhi TY37 (pWR105::R386).

### Figure 2

**Restriction maps of S. sonnei rfb/rfc clones pSSVI109 and pSSVI113.** Restriction maps correspond to insert DNA. Restriction sites belonging to the vector polylinker sequence are labeled with a square. The heavy line indicates the rfb/rfc locus. Scales next to the maps give the DNA length in base pairs (bp).

### Figure 3

**Restriction maps of the** **S. sonnei rfb/rfc** **clone pSSVI165.** The restriction map corresponds to the whole clone including pGB2 vector sequence (black area). The open box within pGB2 sequence labels plasmid replication origin. Otherwise, like for Fig. 2.

### Figure 4

**Restriction maps of rfa**_{**R1**} **clones pSSVI106, 107, 123, 125, and 128.** Restriction maps correspond to insert DNA. The black and hatched area in the map of pSSVI128 define the rfa_{R1} locus (see text). Otherwise, like for Fig. 2.

### Figure 5

**Restriction maps and expression levels in E. coli K-12 of different subclones derived from** **rfa**_{**R1**} **clones pSSVI106 and 107.** Restriction maps correspond to insert DNA. Expression levels of LPS reacting with MAb SH9R are given on the right of the respective maps and are codified from ++++ (strong reaction) to - (no reaction).

### Figure 6

**Restriction map of the** **rfa**_{**sonnei**} **clone pSSVI250.** The restriction map corresponds to insert DNA. Otherwise, like for Fig. 2.

### Figure 7

**Expression of the S. sonnei rfb/rfc clone pSSVI109 in heterologous genetic backgrounds.** Silver staining (A) of LPS minipreparations run on a 15% SDS-PAGE gel and immunoblot analysis using Sh5S MAb (B). Lanes: a, S. sonnei 482-79(pWR105); b, S. sonnei 53GII LPS maxiprep (MP); c, 53GII (pSSVI109); d, 53GII (pSSVI109) LPS MP; e, E. coli K-12 HB101; f, HB101 (pSSVI109); g, HB101 (pSSVI109) LPS MP; h, S. typhi TY21a; i, TY21a (pSSVI109); j, TY21a (pSSVI109) LPS MP; k, V. cholerae CVD103-HgR; l, CVD103-HgR (pSSVI109); m, CVD103-HgR (pSSVI109) LPS MP; n, V. cholerae CVD110; o, CVD110 (pSSVI109).

### Figure 8

**Expression of the S. sonnei rfb/rfc locus in** **E****.** **coli** **K-12 strain SF874** **Drfb/rfc****.** Silver staining (A) of LPS minipreparations run on a 15% SDS-PAGE gel and immunoblot analysis using Sh5S MAb (B). Lanes: a, HB101; b, SF874; c, HB101 (pSSVI109); d, SF874 (pSSVI109); e, HB101 (pSSVI114); f, SF874 (pSSVI114); g, DH5a (pSSVI161); h, SF874 (pSSVI161); i, DH5a (pSSVI165); j, SF874 (pSSVI165).

### Figure 9

**Expression of rfa clones encoding R1-type LPS in heterologous genetic backgrounds.** Silver staining (A) of LPS minipreparations run on a 15% SDS-PAGE gel and immunoblot analysis (B) using Sh9R MAb. Lanes: a and t, pre-stained low molecular weight protein standard (Bio-Rad); b, E. coli K-12 DH5a; c, E. coli K-12 HB101 (pSSVI106); d, HB101 (pSSVI107); e, DH5a (pSSVI107-5); f, HB101 (pSSVI123); g, E. coli K-12 S17.1 (pSSVI250); h, S. typhi TY21a; i, TY21a (pSSVI106); j, TY21a (pSSVI107); k, TY21a (pSSVI107-5); l, TY21a (pSSVI123); m, TY21a (pSSVI250); n, V. cholerae CVD103-HgR; o, CVD103-HgR (pSSVI106); p, CVD103-HgR (pSSVI107); q, CVD103-HgR (pSSVI107-5); r, CVD103-HgR (pSSVI123); s, CVD103-HgR (pSSVI250).

### Figure 10

**Co-expression of** **S.** **sonnei** **rfb****/rfc locus and rfa loci encoding R1-type LPS in heterologous genetic backgrounds.** Silver staining (A) of LPS minipreparations run on a 15% SDS-PAGE gel and immunoblot analysis using Sh5S (B) and Sh9R (C) MAb, respectively. Lanes: a and r, pre-stained low molecular weight protein standard (Bio-Rad); b and q, E. coli K-12 DH5a (pSSVI165); c, S. typhi TY21a; d, TY21a (pSSVI123); e, TY21a (pSSVI165); f, TY21a (pSSVI123/pSSVI165); g, TY21a (pSSVI250); h, TY21a (pSSVI250/pSSVI165); i, V. cholerae CVD103-HgR; j, CVD103-HgR (pSSVI123); k, CVD103-HgR (pSSVI128); l, CVD103-HgR (pSSVI165); m, CVD103-HgR (pSSVI123/pSSVI165); n, CVD103-HgR (pSSVI128/pSSVI165); o, CVD103-HgR (pSSVI250); p, CVD103-HgR (pSSVI250/pSSVI165).

### Figure 11

**Restriction maps of S. typhi H**_{**2**}**S clones pSSVI120 and pSSVI122.** Restriction maps correspond to the whole clones including pUC18 vector sequence (black area). The open box within pUC18 sequence labels the plasmid origin of replication. Ap^{R}, ampicillin resistance genetic determinant. Otherwise like for Figure 2.

### Figure 12

**Restriction map of integration vectors pSSVI154 (S. typhi H**_{**2**}**S locus).** The restriction map corresponds to the whole clone including pMAK700oriT vector sequence (black area). (H/K), (H/H), blunted KpnI, respectively HindIII, ends cloned into blunted HindIII site of pMAK700oriT. oriT, conjugation origin; rep101ts, genetic determinant of the temperature-sensitive replication protein; Cm^{R}, chloramphenicol resistance marker. Otherwise like for Figure 2.

### Figure 13

**Restriction maps of the S. typhi ilv clones pILV12 and pILV18.** Restriction maps correspond to the whole clones, including pUC18 vector sequence (black area). Otherwise like for Figures 2 and 11.

### Figure 14

**Restriction maps of integration vector pSSVI50 (S. typhi ilv locus).** Restriction map corresponds to the whole clone including pMAK700oriT vector sequence (black area). (E/H), (H/H), blunted EcoRI, respectively HindIII, ends cloned into blunted HindIII site of pMAK700oriT. Otherwise like for Figures 2 and 12.

### Figure 15

**Restriction maps of hlyA-****mer** **clone pJMK10 and of integration vector pSSVI155.1 (V. cholerae hlyA locus).** Restriction maps correspond to the whole clones including pUC19, respectively pMA700oriT, vector sequences (black area). The open box within pUC19 sequence corresponds to the plasmid replication origin. (H/P), blunted PstI ends cloned into the blunted HindIII site of pMAK700oriT; hlyA, disrupted hlyA genetic locus; mer, mercury resistance genetic determinant. Otherwise like for Figures 2 and 12.

### Figure 16

**Restriction map of the integration clone pSSVI190 (V. cholerae hlyA locus).** The restriction map corresponds to the whole clone including pMAK700oriT vector sequence (black area). The heavy line indicates the S. sonnei rfb/rfc locus. Otherwise like for Figures 2 and 12.

## Claims

1. A recombinant live vaccine strain for the immunization against gram-negative enteric pathogens displaying the following features:
(a) it is a bacterial strain known to be safe and immunogenic when orally administered to humans;
(b) it carries a set of rfa, rfb and rfc genes coding for the enzymes involved in the biosynthesis of lipopolysaccharide core, preferably heterologous lipopolysaccharide core and heterologous O-polysaccharide antigen;
(c) said set of genes is stably expressed by said carrier strain resulting in the formation of an intact immunogenic hybrid lipopolysaccharide composed of the lipid A from the carrier strain covalently attached to said preferably heterologous core and said heterologous O-polysaccharide, wherein said preferably heterologous core and said heterologous O-polysaccharide form an LPS molecule containing a polymerized O-polysaccharide and being essentially indistinguishable from the LPS molecule of the heterologous gram-negative enteric pathogen.

2. The vaccine strain according to claim 1, wherein said set of genes has been separated from non relevant genetic information of the donor strain.

3. The strain according to claim 1 or 2, wherein the carrier strain is an E. coli strain, a strain belonging to the genus Shigella, a S. typhi strain or a V. cholerae strain.

4. The strain according to claim 3, wherein said S. typhi strain is S. typhi TY21a or S. typhi CVD908.

5. The strain according to claim 3, wherein said V. cholerae strain is V. cholerae CVD103, V. cholerae CVD103-HgR or V. cholerae CVD110.

6. The strain according to any one of claims 1 to 5, wherein said set of heterologous genes are either present on a plasmid vector or stably integrated into the chromosome of the carrier strain at an integration site which is known to be non-essential for inducing a protective immune response by the carrier strain.

7. The strain according to claim 6, wherein said set of genes is integrated into an H₂S locus, the ilv locus, the viaB locus, or any of the aro loci of S. typhi.

8. The strain according to claim 6, wherein said set of genes is inserted into the hlyA locus of V. cholerae.

9. The strain according to any one of claims 1 to 8, wherein said rfa, rfb and rfc genes are integrated in tandem into a single chromosomal integration site or independently integrated into individual integration sites according to any one of claims 6 to 8.

10. The strain according to any one of claims 1 to 9 wherein said rfa genes are derived from an E. coli strain and said rfb and rfc genes are derived from the same or different gram-negative enteric bacteria, preferably from the same or different gram-negative enteric pathogens.

11. The strain according to claim 10, wherein the E. coli strain is characterized by an R1-type LPS core structure.

12. The strain according to claim 10, wherein said gram-negative enteric pathogens are Shigella sonnei, Shigella boydii, Shigella dysenteriae or Shigella flexneri, preferably Shigella flexneri serotype IIa, and most preferably Shigella sonnei.

13. The strain according to any one of claims 1 to 10, wherein said rfa, rfb and rfc genes are derived from said gram-negative enteric pathogen, preferably from Shigella sonnei, Shigella boydii, Shigella dysenteriae or Shigella flexneri, preferably from Shigella flexneri serotype IIa, and most preferably from Shigella sonnei.

14. A live vaccine for the immunization against gram-negative enteric pathogens comprising a strain according to any one of claims 1 to 13, optionally in a mixture with a pharmaceutically acceptable excipient and/or buffer, and/or any delivery system designed to deliver said vaccine strain in a viable state to the intestinal tract.

15. The vaccine according to claim 14, wherein the route of immunization is the oral route.

16. A method for the preparation of the vaccine of claim 14 or 15 which comprises formulating the strain in a mixture with a pharmaceutically acceptable excipient and/or buffer and/or any delivery system designed to deliver said vaccine strain in a viable state to the intestinal tract.

## Patentansprüche

1. Rekombinanter Lebendimpfstoff-Stamm für die Immunisierung gegen gram-negative enterale Pathogene, der die folgenden Merkmale aufweist:
(a) er ist ein bakterieller Stamm, von dem bekannt ist, daß er sicher ist und immunogen wirkt, wenn er oral an Menschen verabreicht wird;
(b) er trägt einen Satz von rfa-, rfb- und rfc-Genen, die Enzyme codieren, die an der Biosynthese der Lipopolysaccharid-Kerneinheit, vorzugsweise der heterologen Lipopolysaccharid-Kerneinheit, und des heterologen O-Polysaccharidantigens beteiligt sind;
(c) dieser Satz von Genen wird von dem Trägerstamm stabil exprimiert, wodurch es zur Bildung eines intakten immunogenen Hybrid-Lipopolysaccharids kommt, das zusammengesetzt ist aus dem Lipid A des Trägerstamms, kovalent gebunden an die vorzugsweise heterologe Kerneinheit und das heterologe O-Polysaccharid, worin die vorzugsweise heterologe Kerneinheit und das heterologe O-Polysaccharid ein LPS-Molekül bilden, das ein polymerisiertes O-Polysaccharid enthält und im wesentlichen nicht unterscheidbar ist von dem LPS-Molekül des heterologen gram-negativen enteralen Pathogens.

2. Impfstoffstamm nach Anspruch 1, worin der Satz von Genen getrennt wurde von nicht relevanter genetischer Information des Donorstammes.

3. Stamm nach Anspruch 1 oder 2, worin der Trägerstamm ein E. coli-Stamm, ein zur Gattung Shigella gehörender Stamm, ein S. typhi-Stamm oder ein V. cholerae-Stamm ist.

4. Stamm nach Anspruch 3, worin der S. typhi-Stamm S. typhi TY21a oder S. typhi CVD908 ist.

5. Stamm nach Anspruch 3, worin der V. cholerae-Stamm V. cholerae CVD103, V. cholerae CVD103-HgR oder V. cholerae CVD110 ist.

6. Stamm nach einem der Ansprüche 1 bis 5, worin der Satz heterologer Gene entweder auf einem Plasmid-Vektor vorliegt oder stabil integriert in das Chromosom des Trägerstammes an einer Integrationsstelle, von der bekannt ist, daß sie nicht essentiell ist für die Induktion einer Immunabwehrreaktion durch den Trägerstamm.

7. Stamm nach Anspruch 6, worin der Satz von Genen in einen H₂S-Locus, den ilv-Locus, den viaB-Locus, oder einen der aro-Loci von S. typhi integriert ist.

8. Stamm nach Anspruch 6, worin der Satz von Genen in den hlyA-Locus von V. cholerae integriert ist.

9. Stamm nach einem der Ansprüche 1 bis 8, worin die rfa-, rfb- und rfc-Gene in Tandemanordnung in eine einzige chromosomale Integrationsstelle integriert sind oder unabhängig in individuelle Integrationsstellen gemäß einem der Ansprüche 6 bis 8 integriert sind.

10. Stamm nach einem der Ansprüche 1 bis 9, worin die rfa-Gene von einem E. coli-Stamm stammen und die rfb- und rfc-Gene von demselben oder anderen gram-negativen Enterobakterien, vorzugsweise von demselben oder anderen gram-negativen enteralen Pathogenen.

11. Stamm nach Anspruch 10, worin der E. coli-Stamm durch eine LPS-Kerneinheit mit der Struktur vom R1-Typ gekennzeichnet ist.

12. Stamm nach Anspruch 10, worin die gram-negativen enteralen Pathogene Shigella sonnei, Shigella boydii, Shigella dysenteriae oder Shigella flexneri sind, vorzugsweise Shigella flexneri Serotyp IIa und besonders bevorzugt Shigella sonnei.

13. Stamm nach einem der Ansprüche 1 bis 10, worin die rfa-, rfb- und rfc-Gene von dem gram-negativen enteralen Pathogen stammen, vorzugsweise von Shigella sonnei, Shigella boydii, Shigella dysenteriae oder Shigella flexneri, vorzugsweise von Shigella flexneri Serotyp IIa, und besonders bevorzugt von Shigella sonnei.

14. Lebendimpfstoff für die Immunisierung gegen gram-negative enterale Pathogene umfassend einen Stamm nach einem der Ansprüche 1 bis 13, gegebenenfalls im Gemisch mit einem pharmazeutisch verträglichen Excipienten und/oder Puffer, und/oder einem Verabreichungssystem, das derart ausgestaltet ist, daß es die Verabreichung des Impfstoff-Stammes in lebendem Zustand in den Darmtrakt erlaubt.

15. Impfstoff nach Anspruch 14, worin der Immunisierungsweg der orale Weg ist.

16. Verfahren zur Herstellung des Impfstoffs nach Anspruch 14 oder 15, umfassend die Formulierung des Stammes in einem Gemisch mit einem pharmazeutisch verträglichen Excipienten und/oder Puffer und/oder einem Verabreichungssystem, das derart ausgestaltet ist, daß es die Verabreichung des Impfstoff-Stammes in lebendem Zustand in den Darmtrakt erlaubt.

## Revendications

1. Souche de vaccin vivant recombinante pour l'immunisation contre des agents pathogènes entériques Gram négatifs présentant les caractéristiques suivantes :
(a) c'est une souche bactérienne connue comme étant sans danger et immunogène lorsque administrée oralement à des êtres humains;
(b) elle porte une série de gènes rfa, rfb et rfc codant pour les enzymes impliquées dans la biosynthèse de noyau de lipopolysaccharide, avantageusement de noyau de lipopolysaccharide hétérologue et d'antigène d'O-polysaccharide hétérologue;
(c) cette série de gènes est exprimée de façon stable par la souche porteuse précitée conduisant à la formation d'un lipopolysaccharide hybride immunogène intact composé du lipide A provenant de la souche porteuse attaché de façon covalente au noyau avantageusement hétérologue et au O-polysaccharide hétérologue précités, le noyau avantageusement hétérologue et le O-polysaccharide hétérologue formant une molécule de LPS contenant un O-polysaccharide polymérisé et étant essentiellement indiscernable de la molécule de LPS de l'agent pathogène entérique Gram négatif hétérologue.

2. Souche de vaccin suivant la revendication 1, dans laquelle la série de gènes a été séparée de l'information génétique non relevante de la souche donneuse.

3. Souche suivant l'une ou l'autre des revendications 1 et 2, dans laquelle la souche porteuse est une souche d'E. coli, une souche appartenant au genre Shigella, une souche de S. typhi ou une souche de V. cholerae.

4. Souche suivant la revendication 3, dans laquelle la souche de S. typhi est la S. typhi TY21a ou S. typhi CVD908.

5. Souche suivant la revendication 3, dans laquelle la souche de V. cholerae est la V. cholerae CVD103, V. cholerae CVD103-HgR ou V. cholerae CVD110.

6. Souche suivant l'une quelconque des revendications 1 à 5, dans laquelle la série de gènes hétérologues précitée est soit présente sur un vecteur plasmidique soit intégrée de façon stable dans le chromosome de la souche porteuse à un site d'intégration qui est connu comme étant non essentiel pour l'induction d'une réponse immunitaire protectrice par la souche porteuse.

7. Souche suivant la revendication 6, dans laquelle la série de gènes précitée est intégrée dans un locus H₂S, le locus ilv, le locus viaB ou l'un quelconque des loci aro de S. typhi.

8. Souche suivant la revendication 6, dans laquelle la série de gènes précitée est insérée dans le locus hlyA de V. cholerae.

9. Souche suivant l'une quelconque des revendications 1 à 8, dans laquelle les gènes rfa, rfb et rfc sont intégrés en tandem dans un seul site d'intégration chromosomique ou intégrés indépendamment dans des sites d'intégration individuels suivant l'une quelconque des revendications 6 à 8.

10. Souche suivant l'une quelconque des revendications 1 à 9, dans laquelle les gènes rfa proviennent d'une souche d'E. coli et les gènes rfb et rfc proviennent des mêmes ou de différentes bactéries entériques Gram négatives, avantageusement des mêmes ou de différents agents pathogènes entériques Gram négatifs.

11. Souche suivant la revendication 10, dans laquelle la souche d'E. coli est caractérisée par une structure de noyau LPS du type R1.

12. Souche suivant la revendication 10, dans laquelle les agents pathogènes entériques Gram négatifs susdits sont Shigella sonnei, Shigella boydii, Shigella dysenteriae ou Shigella flexneri, avantageusement Shigella flexneri, sérotype IIa et le plus avantageusement Shigella sonnei.

13. Souche suivant l'une quelconque des revendications 1 à 10, dans laquelle les gènes rfa, rfb et rfc proviennent de l'agent pathogène entérique Gram négatif susdit, avantageusement de Shigella sonnei, Shigella boydii, Shigella dysenteriae ou Shigella flexneri, avantageusement de Shigella flexneri, sérotype IIa et le plus avantageusement de Shigella sonnei.

14. Vaccin vivant pour l'immunisation contre des agents pathogènes entériques Gram négatifs comprenant une souche suivant l'une quelconque des revendications 1 à 13, éventuellement en un mélange avec un excipient et/ou tampon pharmaceutiquement acceptable et/ou tout système de distribution conçu pour distribuer la souche de vaccin à l'état viable dans le tractus intestinal.

15. Vaccin suivant la revendication 14, dans lequel la voie d'immunisation est la voie orale.

16. Procédé de préparation du vaccin de la revendication 14 ou 15, qui comprend la formulation de la souche en un mélange avec un excipient et/ou tampon pharmaceutiquement acceptable et/ou tout système de distribution conçu pour distribuer la souche de vaccin à l'état viable dans le tractus intestinal.
